# FASCICULE DE BREVET EUROPEEN

(11) **EP 3 894 446 B1**
(45) Date de publication et mention de la délivrance du brevet: **28.12.2022**
(21) Numéro de dépôt: 20706565.7
(22) Date de dépôt: 14.01.2020
(51) Int. Cl.: C02F 1/28, C08L 5/16, C01B 13/00, C08B 37/16, A61L 2/20, C02F 1/78

(54) **PROCÉDÉ DE PRÉPARATION D'UN MATÉRIAU SOLIDE DE STOCKAGE DE L'OZONE, LEDIT MATÉRIAU ET SES UTILISATIONS**
VERFAHREN ZUR HERSTELLUNG EINES FESTEN MATERIALS ZUR SPEICHERUNG VON OZON, MATERIAL UND DESSEN VERWENDUNGEN
METHOD FOR PREPARING A SOLID MATERIAL FOR STORING OZONE, THE MATERIAL AND THE USES THEREOF

(30) Priorité: 14.01.2019 FR 1900325
(43) Date de publication de la demande: 20.10.2021
(73) Titulaire: Institute National Polytechnique de Toulouse, B.P. 34 038 31029 Toulouse Cedex 4 (FR); Ecole d'Ingenieurs de Purpan, 31076 Toulouse Cedex 3 (FR); UNIVERSITE PAUL SABATIER TOULOUSE III, 31062 Toulouse Cedex 9 (FR); CENTRE NATIONAL DE LA RECHERCHE SCIENTIFIQUE (CNRS), 75016 Paris (FR); Institut national de recherche pour l'agriculture, l'alimentation et l'environnement, 75007 Paris (FR)
(72) Inventeur: TORRE, Jean-Philippe, 31432 Toulouse Cedex 4 (FR); PAGES-HOMS, Marielle, 31076 Toulouse Cedex 3 (FR); VIOLLEAU, Frédéric, 31432 Toulouse Cedex 4 (FR); MANERO, Marie-Hélène, 31432 Toulouse Cedex 4 (FR); RICHARD, Romain, 31432 Toulouse Cedex 4 (FR)
(74) Mandataire: Brevalex
(86) Numéro de dépôt international: PCT/FR2020/050038
(87) Numéro de publication internationale: WO 2020/148497

(56) Documents cités:
- JP-A- 2007 210 881
- DETTMER ADAM ET AL: "Stabilization and prolonged reactivity of aqueous-phase ozone with cyclodextrin", CONTAMINANT HYDROLOGY, ELSEVIER, AMSTERDAM, NL, vol. 196, 5 décembre 2016 (2016-12-05), pages 1-9, XP029875084, ISSN: 0169-7722, DOI: 10.1016/J.JCONHYD.2016.11.003 cité dans la demande
- IZUMI KATSUYUKI ET AL: "A porous glass-based ozone sensing chip impregnated with potassium iodide and [alpha]-cyclodextrin", SENSORS AND ACTUATORS B: CHEMICAL, vol. 241, 11 October 2016 (2016-10-11), - 11 October 2016 (2016-10-11), pages 116-122, XP029864099, ISSN: 0925-4005, DOI: 10.1016/J.SNB.2016.10.026

## Description

### Domaine technique

La présente invention concerne le domaine technique général du stockage de l'ozone.

Plus particulièrement, la présente invention propose un procédé et une installation permettant de préparer un matériau se présentant sous forme solide et, de fait, facilement manipulable, dans lequel de l'ozone est stocké.

La présente invention concerne également ce matériau de stockage d'ozone, qui permet d'augmenter la stabilité de l'ozone et donc sa durée de vie, ainsi que l'utilisation de ce matériau dans toutes les applications visant à mettre à profit les propriétés de l'ozone.

### État de la technique antérieure

Du fait de son fort pouvoir oxydant, l'ozone est utilisé actuellement pour de nombreuses applications, comme, par exemple, pour la désinfection et le traitement de l'eau. L'utilisation d'ozone est également regardée attentivement pour des applications potentielles telles que le traitement des végétaux.

Pour autant, et même si certains sites commerciaux affichent cette possibilité de traitement, l'application de l'eau enrichie en ozone dans un milieu ouvert est peu pertinente. En effet, la gouttelette d'eau enrichie se retrouvant dans une atmosphère complètement dépourvue d'ozone, va être au cœur d'un phénomène de désorption : l'ozone va sortir très rapidement de la goutte d'eau et ne parviendra donc pas jusqu'à sa cible (la feuille si on traite une plante).

Par ailleurs, l'ozone est un gaz en conditions ambiantes qui ne peut être stocké du fait de son instabilité. Ainsi, cette molécule doit être produite au besoin par un ozoneur, appareil aussi appelé « générateur d'ozone », qui produit une décharge électrique dans un flux de dioxygène (O₂) et qui permet de créer la molécule d'ozone (O₃). L'ozone peut également être produit par d'autres procédés impliquant un plasma ou une lumière UV notamment à la longueur d'ondes de 185 nm. Cette molécule d'ozone a une durée de vie limitée, de l'ordre d'une vingtaine de minutes dans l'eau en conditions ambiantes. Elle ne peut pas être stockée par exemple dans une bouteille à l'état pur ou en mélange. A noter que les risques d'explosion sont élevés lorsque le mélange envisagé est un mélange de dioxygène et d'ozone dans lequel la concentration en ozone est supérieure à 10-13% mol et/ou lorsque ce mélange est comprimé à une pression de l'ordre de plusieurs dizaines de bars. On peut prolonger la durée de vie de l'ozone, par exemple en abaissant fortement la température (azote liquide par exemple), mais il n'existe, à l'heure actuelle, aucun système pouvant stocker de façon durable une grande quantité d'ozone.

Cette limitation de stockage pose des problèmes pour utiliser facilement ce gaz car il faut obligatoirement un ozoneur sur place et, si tel est le cas, le gaz produit doit être utilisé immédiatement. De plus, en fonction des applications visées, la nécessité d'avoir un ozoneur sur place peut être considérée comme trop chère, trop encombrante ou encore trop technique.

La très faible durée de vie de l'ozone est liée au fait que la molécule d'ozone est instable : lorsque deux molécules d'ozone se rencontrent, elles peuvent se décomposer en trois molécules de dioxygène selon la réaction 2 O₃ -> 3 O₂. Cette décomposition est favorisée par la température et la présence d'éléments catalyseurs comme certains matériaux solides, certaines molécules ou certaines conditions d'humidité. Ainsi, la durée de demi-vie de l'ozone dans l'air est théoriquement de 3 jours mais, comme ce gaz réagit avec quasiment tous les matériaux environnants, elle est en pratique de quelques secondes. Il est donc très difficile de limiter la réaction de décomposition de l'ozone, à part à très basses températures où le mouvement des molécules est réduit. Par ailleurs, la pressurisation d'ozone conduit à augmenter son instabilité (rapprochement des molécules) et donc à réduire sa durée de vie.

Plusieurs stratégies ont déjà été proposées pour résoudre le problème technique du stockage de l'ozone.

Des recherches menées dans les années 60 ont proposé une voie théorique de stockage de l'ozone liquide, par stabilisation par du fluor, du trifluorure de chlore et de l'acide nitrique, mais ces recherches n'ont pas donné de suite, probablement en raison de la dangerosité de ces produits. Le stockage de mélange O₂/O₃ liquide sous forte pression a également été envisagé et testé mais jamais commercialisé car extrêmement dangereux. En effet, il existe des risques d'explosion si le pourcentage d'ozone liquide est trop fort et/ou si le dioxygène s'évapore et crée une atmosphère explosive. De plus, les risques de décomposition thermique de l'ozone ajoutés aux risques de décomposition au contact de solides ou molécules ont conduit les équipes de recherche impliquées à abandonner ces thématiques.

La société Air Liquide a commercialisé quelque temps un stockage d'ozone liquide plus sûr, dans du liquide cryogénique (Fréon). Mais la difficulté à séparer les deux gaz et les problèmes de décomposition de l'ozone ont définitivement mis fin à la commercialisation de ce produit.

Le stockage d'ozone dans un hydrate de gaz a également été proposé. Ce concept a été montré, pour la première fois, en 1964 par McTurk et Waller qui sont parvenus à former un hydrate mixte tétrachlorométhane (CCl₄) + O₃, en saturant une solution de CCl₄ avec de l'ozone à -2°C. Toutefois, le CCl₄ est un produit extrêmement toxique non envisageable dans nombre des applications utilisant l'ozone comme oxydant ou comme désinfectant **[1].** Les hydrates mixtes contenant de l'ozone formé en mettant en contact un mélange O₂/O₃ et un gaz annexe (« help gas ») ont été étudiés essentiellement par une équipe japonaise (Ohmura et Mori) entre 2010 et 2014. Parmi les travaux publiés par cette équipe, on peut citer Nakagima et *al,* 2012 qui étudie l'équilibre de phase et la composition de l'hydrate mixte O₂/O₃ + CO₂ **[2].** Il a été montré dans ce travail qu'un mélange O₂/O₃ contenant 10-12 % mol en O₃ mélangé à du CO₂ dans un rapport molaire 1/7 formé à la pression de 19 bar et à une température de 0,1°C est stable en conditions aérées (tube ouvert à l'air) durant plus de 20 jours à -25°C et peut stocker une quantité d'ozone de 0,1% massique.

A l'heure actuelle, l'eau ozonée et la glace ozonée peuvent être utilisées. Ainsi, la demande de brevet JP 2007/210881 au nom de Kurita Water Ind. Ltd., publiée le 23 août 2007 **[3]** propose un procédé pour préparer une glace ozonée. Mais, la concentration d'ozone dans l'eau ozonée et dans la glace ozonée reste très faible avec une durée de vie assez courte.

Afin d'augmenter la durée de vie de l'ozone dans l'eau ozonée et d'obtenir une réactivité prolongée, il a été proposé de piéger, en phase aqueuse, de l'ozone dans une cyclodextrine du type hydroxypropyl-β-cyclodextrine **[4].** De même, la demande de brevet US 2018/0178263 au nom de OXYTEC LLC, publiée le 28 juin 2018 **[5]** propose un procédé pour réduire la contamination dans l'eau ou le sol, consistant à injecter une solution aqueuse d'un clathrate d'ozone utilisant un oligosaccharide cyclique tel qu'une cyclodextrine. Dans ces documents, la cyclodextrine est toujours mise en solution dans l'eau puis l'ozone sous forme gazeuse est injecté dans cette solution.

Dans un aspect distinct du stockage de l'ozone, la demande de brevet US 2016/0367967 au nom de Temple University of the Commonwealth System of Higher Education, publiée le 22 décembre 2016 **[6]** concerne des systèmes et procédés pour régénérer un adsorbant du type β-cyclodextrine après son utilisation dans un procédé de décontamination de l'eau. Le procédé de régénération est basé sur le traitement de la β-cyclodextrine avec de l'ozone gazeux utilisé pour détruire les contaminants piégés dans la cavité de la β-cyclodextrine. L'adsorbant β-cyclodextrine utilisé peut être régénéré et réutilisé pour de nombreux cycles de traitement grâce à ce processus d'ozonation, en phase liquide.

Les inventeurs se sont fixé pour but de mettre au point un matériau facile à préparer, facile à utiliser et pouvant stocker l'ozone en grande quantité et de façon durable de façon à lever un certain nombre de verrous quant à l'utilisation pratique de ce gaz.

### Présentation de l'invention

La présente invention permet d'atteindre le but que se sont fixé les inventeurs et de résoudre tout ou partie des inconvénients des procédés et matériaux de stockage de l'ozone de l'état de la technique.

En effet, les inventeurs ont mis au point un procédé permettant de produire un matériau de stockage de l'ozone se présentant sous forme solide et donc facilement manipulable et utilisable. Le procédé de préparation selon l'invention permet d'obtenir une poudre qui peut être utilisée telle quelle par saupoudrage, sous forme d'une solution, dispersion, émulsion ou suspension par pulvérisation, ou encore sous forme plus compactée notamment pour en faire des pastilles.

Par ailleurs, le matériau solide de stockage préparé conformément au procédé selon l'invention permet de stocker, de façon durable, de l'ozone puisque les tests non optimisés réalisés par les inventeurs ont montré que l'ozone peut y être stocké sur plusieurs jours à température ambiante, ce qui correspond à une amélioration significative eu égard à la demi-vie de l'ozone, à température ambiante, qui est de l'ordre de la vingtaine de minutes dans l'eau ozonée. Il s'agit donc non seulement d'un matériau de stockage de l'ozone mais aussi d'un matériau de stabilisation de l'ozone. Ce matériau offre également une grande capacité potentielle de stockage de l'ozone. Certains des matériaux décrits dans la partie expérimentale ci-après ont une capacité de stockage 400 fois supérieure à celle de l'eau ozonée et même 800 fois supérieure à celle de l'eau ozonée.

Le fait que le matériau solide de stockage préparé conformément au procédé selon l'invention soit facilement manipulable tout en garantissant la stabilisation de l'ozone sur du long terme permet de dissocier le lieu de production du lieu d'utilisation voire même d'envisager un lieu de stockage distinct du lieu de production et du lieu d'utilisation. Le matériau de stockage de l'ozone selon l'invention constitue donc également un matériau de transport de l'ozone.

Tous ces avantages sont obtenus en mettant en œuvre un procédé simple, facilement industrialisable, ne nécessitant pas de conditions opératoires à risque et utilisant des matières premières du type cyclodextrines ou dérivés de cyclodextrines facilement accessibles et relativement bon marché. En effet, les cyclodextrines, déjà largement utilisées dans les formulations cosmétiques et pharmaceutiques, constituent un produit naturel, non dangereux, à base de sucres, éco-compatible et se dégradant dans le milieu naturel. L'utilisation de dérivés de cyclodextrines susceptibles de présenter des propriétés différentes notamment en termes de solubilité, hydrophilie, hydrophobie, propriétés de complexation, ... permet d'envisager la préparation d'un matériau solide de stockage de l'ozone modulable et polyvalent, adapté à des utilisations ciblées. Autre fait intéressant, la présence de cyclodextrines ou de dérivés de cyclodextrines peut permettre d'obtenir un matériau solide de stockage de l'ozone coulant dans l'eau et libérant l'ozone au sein du fluide, du fait de la densité des cyclodextrines ou des dérivés de cyclodextrines supérieure à celle de l'eau. Ceci est un avantage additionnel si, par exemple, la poudre est conditionnée sous forme de pastilles.

Plus particulièrement, la présente invention concerne un procédé de préparation d'un matériau solide de stockage de l'ozone comprenant la mise en contact de cyclodextrines et/ou de dérivés de cyclodextrines se présentant sous forme solide avec un gaz comprenant de l'ozone moyennant quoi un matériau solide de stockage de l'ozone est obtenu.

Par « cyclodextrine » on entend un oligosaccharide cyclique de formule (C₆H₁₀O₅)ₙ composé de n sous-unités de glucopyranose de formule C₆H₁₀O₅ liées en α-(1,4) avec n représentant un nombre entier. Les termes « cyclodextrine », « cycloamylose », « cycloglucane », « cyclomaltooside » et « dextrine de Schardinger » sont équivalents et utilisables de façon interchangeable.

Les cyclodextrines mises en œuvre dans le cadre de l'invention présentent une structure annulaire, formant une cage en forme de cône tronqué délimitant une cavité dont la taille est dépendante du nombre n de sous-unités de glucopyranose et qui peut stabiliser d'autres molécules, où n est avantageusement compris entre 6 et 35.

A titre d'exemples particuliers de cyclodextrines utilisables dans le cadre de la présente invention, on peut citer les cyclomaltohexaoses, cyclohexaamylose, α-cycloamylases ou α-cyclodextrines (a-CD) dans lesquelles n représente 6, les cyclomaltoheptaoses ou β-cyclodextrines (β-CD) dans lesquelles n représente 7 et les cyclomaltooctaoses ou γ-cyclodextrines (y-CD) dans lesquelles n représente 8, les cyclomaltononaoses dans lesquelles n est égal à 9, les cyclomaltoheneicosaoses dans lesquelles n est égal à 21, les cyclomaltodoicosaoses dans lesquelles n est égal à 22 et les cyclomaltohentricontaoses dans lesquelles n est égal à 31.

Par « dérivé de cyclodextrine », on entend une cyclodextrine telle que précédemment définie, modifiée chimiquement, réticulée, immobilisée et/ou organisée en superstructure moléculaire. Quelle que soit la variante envisagée, un dérivé de cyclodextrine mis en œuvre dans l'invention présente toujours une cavité apte à stabiliser d'autres molécules.

Un dérivé du type cyclodextrine modifiée chimiquement est obtenu en substituant au moins un atome d'hydrogène et/ou au moins un radical hydroxyle d'une cyclodextrine telle que précédemment définie par un atome ou un groupement chimique tel qu'un atome d'halogène, un groupement alkyle, un groupement hydroxyalkyle, un groupement thioalkyle, groupement sulfhydryle, un groupement acétyle, un groupement silyle, un groupement acyle, un groupement sulfonyle, un groupement amine, un groupement sulfoalkyléther, un groupement sulfate, un groupement phosphate, un groupement carboxyle, un groupement carboxylester, un groupement ammonium quaternaire, un groupement glucosyle, un groupement maltosyle, un groupement chlorotriazinyle ou un groupement ammonium quaternaire. En fonction de la nature chimique du ou des groupement(s) substituant(s) mis en œuvre, le dérivé de cyclodextrine peut être ionique ou amphiphile.

A titre d'exemples illustratifs et non limitatifs de dérivés du type cyclodextrine modifiée chimiquement, on peut citer une α-CD, une β-CD ou une γ-CD méthylée de façon aléatoire ; une méthyl-α-CD ; une méthyl-β-CD ; une méthyl-γ-CD ; la heptakis(2,3,6-tri-O-methyl)-β-CD ; une α-CD, une β-CD ou une γ-CD faiblement méthylée en position 2 (2-O-méthylée) ; une α-CD, une β-CD ou une γ-CD diméthylée ; une α-CD, une β-CD ou une γ-CD perméthylée ; une α-CD, une β-CD ou une γ-CD perpentylée ; une α-CD, une β-CD ou une γ-CD acétylée ; une α-CD, une β-CD ou une γ-CD peracétylée ; une α-CD, une β-CD ou une γ-CD hydroxypropylée ; une α-CD, une β-CD ou une γ-CD hydroxyéthylée ; une α-CD, une β-CD ou une γ-CD sulfatée ; une α-CD, une β-CD ou une y-CD phosphatée ; une α-CD, une β-CD ou une γ-CD carboxyméthylée ; une α-CD, une β-CD ou une y-CD carboxyméthyléthérée ; une 3-triméthylammonium-2-hydroxypropyl-éther-α-CD ; une 3-triméthylammonium-2-hydroxypropyl-éther-β-CD ; une 3-triméthylammonium-2-hydroxypropyl-éther-γ-CD; la mono-(6-mercapto-6-déoxy)-β-CD; la mono-(6-amino-6-déoxy)-β-CD; la heptakis(6-amino-6-déoxy)-β-CD ; la mono-(6-(diéthylènetriamine)-6-déoxy)-β-CD ; la hexakis-(6-iodo-6-déoxy)-α-CD ; une sulfobutyléther-α-CD ; une sulfobutyléther-β-CD ; une sulfobutyléther-γ-CD; la 3-triméthylammonium-2-hydroxylpropyléther-α-CD ; la 3-triméthylammonium-2-hydroxylpropyléther-β-CD ; la 3-triméthylammonium-2-hydroxylpropyléther-γ-CD ; la glucosyl-α-CD ; la glucosyl-β-CD ; la glucosyl-γ-CD ; la maltosyl-α-CD ; la maltosyl-β-CD ; la maltosyl-γ-CD ; la chlorotriazinyl-α-CD ; la chlorotriazinyl-β-CD ; et la chlorotriazinyl-γ-CD.

Un dérivé du type cyclodextrine réticulée est typiquement obtenu en formant des liaisons entre des cyclodextrines ou des cyclodextrines chimiquement modifiées telles que précédemment définies grâce à un agent réticulant tel que l'épichlorhydrine, le 1,4-butanedioldiglycidyléther, le 1,2-epoxypropane, le 1,3-diglycidylglycérol, le 1,4-phényldiisocyanate, le 2,4-toluène diisocyanate, la glutaraldéhyde ou l'acide citrique. Un dérivé du type cyclodextrine réticulée se présente sous forme d'un polymère soluble ou insoluble tel que des gels ou hydrogels réticulés. Ainsi, les polymères de cyclodextrines sont des exemples de dérivés de cyclodextrine réticulés. Ce type de dérivé peut être préparé en deux étapes avec, tout d'abord, une réticulation des molécules de CD par l'épichlorhydrine en présence d'un autre agent réticulant cationique puis une carboxyméthylation des particules réticulées en surface. A titre d'exemple particulier, on peut citer un gel amphotère de cyclodextrines réticulées par l'épichlorohydrine en présence de 3-chlorure-2-hydroxypropyl triméthylammonium, et carboxyméthylé **[7].**

Un dérivé du type cyclodextrine immobilisée correspond à des cyclodextrines ou des cyclodextrines modifiées chimiquement telles que précédemment définies, greffées sur des polymères comme des polyalkylamines, des polyéthylènes imines, des polyallylamines ou des polyacrylates ; sur des membranes comme des membranes liquides supportées ou des membranes denses ; sur des textiles ; sur des billes inorganiques comme des billes de silice ou de charbon actif ; ou sur des résines organiques.

A titre d'exemples illustratifs et non limitatifs de dérivés du type cyclodextrine immobilisée, on peut citer les membranes de poly(acétate de vinyle)-β-CD réticulé avec du di-époxyde, les membranes mixtes polysiloxane-β-CD sur membrane céramique, les membranes de poly(acétate de vinyle)-α-CD réticulé avec de l'hexaméthylène diisocyantate, les β-CD fixées sur des fibres de polyacrilonitrile ou de polyester, des CD greffées sur du chitosan, des monochlorotriazinyl-β-CD fixées sur des fibres de coton, de coton/polyuréthane ou de coton/polyamide et des fibres de laine, de cellulose ou de poly(téréphtalate d'éthylène) traitées avec le système β-CD/acide 1,2,3,4-butanetétracarboxylique.

A titre d'exemples de dérivés du type cyclodextrine organisée en superstructure moléculaire, on peut citer les polyrotaxanes, les polypseudorotaxanes constitués d'une chaîne de poly(éthylène glycol) constituant le stator complexé par plusieurs cyclodextrines constituant la partie mobile ou rotor et les tubes moléculaires.

Le procédé selon la présente invention envisage de mettre en œuvre (i) un ensemble de cyclodextrines identiques ou différentes, (ii) un ensemble de dérivés de cyclodextrines identiques ou différents ou encore (iii) un ensemble de cyclodextrines, identiques ou différentes et de dérivés de cyclodextrines, identiques ou différents.

Avantageusement, les cyclodextrines et/ou les dérivés de cyclodextrines mis en œuvre dans la présente invention sont choisis dans le groupe constitué par les α-CD, les β-CD, les y-CD, les α-CD hydroxypropylées, les β-CD hydroxypropylées, les γ-CD hydroxypropylées, les α-CD diméthylées, les β-CD diméthylées, les γ-CD diméthylées ; les sulfobutyléther-α-CD, les sulfobutyléther-β-CD, les sulfobutyléther-γ-CD, les α-CD sulfatées, les β-CD sulfatées, les γ-CD sulfatées, les α-CD phosphatées, les β-CD phosphatées, les γ-CD phosphatées ; les α-CD carboxyméthylées, les β-CD carboxyméthylées, les γ-CD carboxyméthylées, les α-CD carboxyméthyléthérées, les β-CD carboxyméthyléthérées, les γ-CD carboxyméthyléthérées, les 3-triméthylammonium-2-hydroxypropyl-éther-α-CD ; les 3-triméthylammonium-2-hydroxypropyl-éther-β-CD ; les 3-triméthylammonium-2-hydroxypropyl-éther-γ-CD ; les dérivés de cyclodextrines réticulés et leurs mélanges.

Les cyclodextrines et/ou les dérivés de cyclodextrines mis en œuvre dans le cadre de l'invention se présentent sous forme solide et notamment sous forme d'une poudre. La granulométrie de cette poudre dépend du type de cyclodextrines et/ou de dérivés de cyclodextrines mis en œuvre. Typiquement, le diamètre moyen des grains de la poudre de cyclodextrines et/ou de dérivés de cyclodextrines est compris entre 10 nm et 10 mm et notamment entre 10 µm et 5 mm.

Les cyclodextrines et/ou les dérivés de cyclodextrines mis en œuvre dans le cadre de l'invention peuvent également se présenter sous forme solide compacte notamment sous forme de bloc solide ou d'assemblage de plusieurs blocs solides.

A l'état natif i.e. soit après leur production naturelle, soit après leur synthèse chimique, les cyclodextrines et/ou les dérivés de cyclodextrines solides mis en œuvre dans le cadre de l'invention sont stabilisés par une ou plusieurs molécule(s) d'eau présente(s) notamment dans leur cavité. Avantageusement, lors de la mise en contact avec le gaz comprenant de l'ozone, les cyclodextrines et/ou les dérivés de cyclodextrines mis en œuvre dans le cadre de l'invention sont exempts de toute molécule différente d'une molécule d'eau et susceptible de réagir avec l'ozone.

Par ailleurs, le procédé selon la présente invention peut présenter une étape préalable à la mise en contact entre les cyclodextrines et/ou les dérivés de cyclodextrines et le gaz comprenant de l'ozone visant à éliminer tout ou partie des molécules d'eau associées aux cyclodextrines et/ou aux dérivés de cyclodextrine. A titre d'exemples, un tel traitement peut être un traitement thermique ou une extraction sous vide. En variante, le procédé selon la présente invention peut présenter une étape préalable à la mise en contact entre les cyclodextrines et/ou les dérivés de cyclodextrines et le gaz comprenant de l'ozone visant à remplacer tout ou partie des molécules d'eau présentes dans les cavités des cyclodextrines et/ou des dérivés de cyclodextrines par une substance non réactive à l'ozone comme du dioxyde de carbone. A titre d'exemples, un tel traitement peut consister à mettre en contact les cyclodextrines et/ou les dérivés de cyclodextrines avec du CO₂ sous pression.

Avantageusement, le gaz comprenant de l'ozone mis en œuvre dans le cadre de la présente invention est un mélange gazeux comprenant de l'ozone et au moins un autre gaz tel que du dioxygène, du dioxyde de carbone, du diazote ou un de leurs mélanges. Lorsque le mélange gazeux comprend de l'ozone et du dioxygène, ce mélange est produit à partir d'un générateur d'ozone ou ozoneur, typiquement alimenté avec de l'air ambiant, de l'air sec, de l'air humide, de l'air compressé ou de l'oxygène pur. La concentration en ozone dans le mélange gazeux en sortie du générateur d'ozone est comprise entre 10 g/Nm³ et 180 g/Nm³. La production d'ozone dans un tel générateur d'ozone ou ozoneur peut impliquer une décharge électrique, un plasma ou une lumière UV notamment à la longueur d'onde de 185 nm.

Typiquement, dans le procédé selon l'invention, la mise en contact entre les cyclodextrines et/ou les dérivés de cyclodextrines et le gaz comprenant de l'ozone est réalisée à une température comprise entre 0°C et 80°C, notamment entre 5°C et 70°C, en particulier, entre 10°C et 60°C, plus particulièrement entre 15°C et 55°C et, plus particulièrement encore, entre 15°C et 40°C. Ainsi, cette mise en contact peut être réalisée à température ambiante. Par « température ambiante », on entend toute température comprise entre 18°C et 28°C. En variante, cette mise en contact peut être réalisée à 50°C.

Typiquement, dans le procédé selon l'invention, la mise en contact entre les cyclodextrines et/ou les dérivés de cyclodextrines et le gaz comprenant de l'ozone dure entre 1 min et 8 h, notamment entre 15 min et 6 h et, en particulier, entre 30 min et 4 h. Plus particulièrement, cette mise en contact peut durer, par exemple, de l'ordre de 1 h (i.e. 1 h ± 15 min), de l'ordre de 2 h (i.e. 2 h ± 15 min) ou de l'ordre de 3 h (i.e. 3 h ± 15 min).

Dans le procédé selon l'invention, la mise en contact entre les cyclodextrines et/ou les dérivés de cyclodextrines et le gaz comprenant de l'ozone peut être réalisée dans tout système permettant une réaction dite « gaz/solide », i.e. dans un appareil ou dispositif permettant de mettre efficacement en contact un gaz avec un solide. Considérant la phase solide correspondant, dans le procédé selon l'invention, aux cyclodextrines et/ou aux dérivés de cyclodextrines sous forme solide, la mise en contact peut être discontinue, semi-continue ou continue.

Dans un procédé discontinu (ou batch), le solide est chargé dans un contacteur gaz/solide, opérant en lit fixe ou fluidisé.

Dans un procédé semi-continu (semi-batch), plusieurs contacteurs opèrent simultanément soit en lit fixe, soit en lit fluidisé. Par un jeu de vannes, il est possible de charger ou décharger un appareil dans lequel la réaction est terminée pendant qu'un autre est en réaction. De cette façon on peut simuler un procédé continu.

Dans un procédé continu fluidisé, le solide peut être expansé dans le contacteur ou peut circuler à contre-courant du flux de gaz réactif. La réaction a lieu lorsque le gaz rencontre les particules solides en mouvement ou non. On peut avoir des lits mobiles, ou des lits fluidisés multi-étages avec recirculation du solide entre les étages.

Dans un lit fixe, les particules solides sont placées dans un réacteur ayant un fond poreux (appelé « distributeur ») capable de laisser passer au travers de ce fond le gaz comprenant de l'ozone de bas en haut tout en retenant les particules placées au-dessus de ce fond. Le lit est dit « fixe » car il ne bouge pas (les solides restent immobiles) l'écoulement du gaz se faisant dans l'espace interstitiel laissé libre entre les particules fixes. Le débit de gaz est, dans ce cas, volontairement faible pour ne pas mettre en mouvement les particules.

Dans un lit fluidisé, le débit de gaz utilisé est plus élevé que dans un procédé à lit fixe. La vitesse de gaz entre les particules solides augmente, et par suite, le frottement du gaz sur la surface des particules augmente également. Lorsque le frottement du gaz crée une force suffisante pour compenser le poids de toutes les particules du lit, on dit que le lit de particules est fluidisé. La vitesse du gaz à partir de laquelle le lit est fluidisé est appelée « vitesse minimale de fluidisation ». Une fois ce passage de l'état de lit fixe au lit fluidisé réalisé par le maintien du passage de gaz au débit ad hoc, la couche de particules fluidisées se comporte comme un liquide où, par exemple, des objets plus légers peuvent flotter à sa surface et où des objets plus lourds peuvent couler.

Dans un lit mobile, le débit de gaz utilisé est encore supérieur à celui utilisé précédemment. Ainsi, les particules peuvent s'écarter les unes des autres laissant plus d'espace libre entre elles et des bulles de gaz peuvent apparaître. Des particules solides peuvent être entraînées par le gaz et quitter le réacteur. La solution envisagée est généralement la mise en place d'un cyclone après le réacteur qui permet de séparer le gaz et les particules solides par force centrifuge. Le solide peut alors être collecté et réinjecté dans le bas du réacteur jusqu'à atteindre le temps de réaction désiré.

Dans le procédé selon l'invention, la mise en contact entre les cyclodextrines et/ou les dérivés de cyclodextrines et le gaz comprenant de l'ozone peut être également réalisée dans des contacteurs tels que des mélangeurs de poudres ou des réacteurs agités. Ces mélangeurs peuvent être regroupés en plusieurs catégories: les cuves tournantes (enceintes fermées mises en rotation), les mélangeurs convectifs (constitués d'une cuve ou enceinte fixe et d'une partie mécanique mobile (agitateur, ruban) à l'intérieur de cette cuve), les mélangeurs à fort cisaillement (cuve statique dans laquelle tourne un agitateur à grande vitesse), les mélangeurs statiques (circulation de la poudre dans une enceinte contenant des internes), les mélangeurs à lits fluidisés (circulation d'un gaz au sein du lit de poudre permettant de la mettre en mouvement au sein de l'appareil), et les appareils constitués d'une combinaison de plusieurs mélangeurs. Ces appareils peuvent fonctionner en mode continu ou discontinu (batch). On peut citer par exemple, pour les mélangeurs discontinus, les mélangeurs dits à vis conique, à pales, à vis à ruban, à socs, birotor à palettes, verticaux, hybrides, etc. Pour les mélangeurs continus, on peut citer par exemple les mélangeurs à fort impact, modulaires, ou rapides à pales.

Comme expliqué ci-dessus, l'homme du métier saura adapter, sans effort inventif, le débit du gaz lors de la mise en contact entre les cyclodextrines et/ou les dérivés de cyclodextrines sous forme solide et le gaz comprenant de l'ozone en fonction des dispositifs ou installations choisi(e)s pour cette mise en contact. Typiquement, le débit de gaz en sortie de la source de gaz comprenant de l'ozone et notamment en sortie de l'ozoneur est compris entre 15 L/h et 1 m³/h.

De la même façon, l'homme du métier saura choisir, sans effort inventif, la quantité de cyclodextrines et/ou de dérivés de cyclodextrines sous forme solide à utiliser en fonction des dispositifs ou installations choisi(e)s pour cette mise en contact.

Le procédé selon la présente invention peut comprendre une étape supplémentaire consistant à récupérer les cyclodextrines et/ou les dérivés de cyclodextrines suite à leur mise en contact avec le gaz comprenant de l'ozone. Cette récupération peut consister à décharger les cyclodextrines et/ou les dérivés de cyclodextrines du réacteur tel que précédemment défini dans lequel la mise en contact a eu lieu.

Le matériau obtenu ou récupéré à l'issue du procédé selon l'invention a la même apparence visuelle que les cyclodextrines et/ou les dérivés de cyclodextrines mis en œuvre lors du procédé. Il se présente donc sous forme de poudre, dont le diamètre moyen des grains est typiquement compris entre 10 nm et 10 mm et notamment entre 10 µm et 5 mm, ou sous forme solide compacte.

Par contre, le matériau obtenu ou récupéré se distingue, chimiquement, du matériau de départ correspondant aux cyclodextrines et/ou aux dérivés de cyclodextrines mis en œuvre lors du procédé i.e. les cyclodextrines et/ou les dérivés de cyclodextrines n'ayant pas été mis en contact avec le gaz contenant de l'ozone. En effet, le matériau obtenu ou récupéré est positif au test découlant du dosage à l'iodure de potassium/thiosulfate, contrairement au matériau de départ. Le matériau obtenu ou récupéré présente aussi des propriétés oxydantes et biocides nettes, que ne possède pas le matériau de départ. De plus, le matériau obtenu ou récupéré libère de l'ozone lors de tests réalisés avec un détecteur d'ozone dans un récipient fermé. En effet, la concentration en ozone augmente, au cours du temps, dans le récipient contenant ce matériau obtenu ou récupéré, alors qu'aucune libération d'ozone n'est observée avec le matériau de départ.

Ces différents éléments associés au fait que le matériau obtenu ou récupéré perde une partie de son activité oxydante dans le temps constituent des preuves que le matériau obtenu ou récupéré contient de l'ozone. En d'autres termes, suite à leur mise en contact avec un gaz comprenant de l'ozone, au moins une partie des cavités des cyclodextrines et/ou des dérivés de cyclodextrines se présentant sous forme solide contient de l'ozone, ce dernier pouvant se trouver sous forme moléculaire, ionique et/ou radicalaire. Le matériau obtenu ou récupéré constitue donc bien un matériau solide de stockage d'ozone.

Par « stockage de l'ozone», on entend, dans le cadre de la présente invention, un stockage physique et/ou chimique de l'ozone. Un stockage physique de l'ozone est un stockage sans modification chimique de l'ozone telle que dissolution, adsorption, complexation, .... Un stockage chimique de l'ozone est un stockage impliquant une réaction chimique, comme, par exemple, une réaction chimique entre l'ozone et les cyclodextrines et/ou les dérivés de cyclodextrines. Ainsi, suite à leur mise en contact avec un gaz comprenant de l'ozone, les cyclodextrines et/ou les dérivés de cyclodextrines se présentant sous forme solide contiennent par exemple de l'ozone (stockage physique) et/ou de l'ozone (stockage chimique) stabilisé par réaction avec les cyclodextrines et/ou les dérivés de cyclodextrines, ou éventuellement par réaction avec des molécules d'eau et/ou avec d'autres espèces présentes dans le gaz comprenant de l'ozone.

La présente invention concerne également une installation susceptible d'être mise en œuvre dans le cadre du procédé de préparation tel que précédemment défini. Cette installation comprend au moins un réacteur contenant des cyclodextrines et/ou des dérivés de cyclodextrines sous forme solide tel(le)s que précédemment défini(e)s en connexion fluidique avec une source d'un gaz comprenant de l'ozone.

Le réacteur de l'installation selon l'invention est notamment un contacteur gaz/solide, opérant en lit fixe ou fluidisé, un mélangeur de poudre ou un réacteur agité et ce, dans toutes les variantes précédemment décrites.

Lorsque le gaz comprenant de l'ozone est un mélange comprenant de l'oxygène et de l'ozone, la source est un générateur d'ozone. Là aussi, toutes les informations précédemment fournies pour caractériser un tel générateur d'ozone s'appliquent également à l'installation selon l'invention.

Avantageusement, l'installation selon l'invention comprend, en outre, un ou plusieurs élément(s) choisi(s) dans le groupe constitué par un filtre, un destructeur d'ozone, un débitmètre, des sondes de température, des analyseurs d'ozone et des vannes. En particulier, l'installation selon l'invention comprend l'ensemble de ces éléments. La partie expérimentale ci-après décrit une forme de mise en œuvre particulière d'une installation selon la présente invention.

La présente invention concerne également un matériau solide de stockage de l'ozone susceptible d'être préparé par un procédé de préparation tel que précédemment défini. Typiquement, le matériau solide de stockage de l'ozone objet de la présente invention comprend des cyclodextrines et/ou des dérivés de cyclodextrines se présentant sous forme solide, dont au moins une partie des cavités contient de l'ozone. Dans une forme de mise en œuvre particulière, le matériau solide de stockage de l'ozone objet de la présente invention est constitué de cyclodextrines et/ou de dérivés de cyclodextrines se présentant sous forme solide, dont au moins une partie des cavités contient de l'ozone.

Tout ce qui a été précédemment décrit en rapport avec les cyclodextrines, les dérivés de cyclodextrine, le matériau obtenu ou récupéré s'applique également à cet aspect de l'invention.

Avantageusement, le matériau solide de stockage de l'ozone selon l'invention se présente sous forme compactée et/ou sous forme conditionnée.

Lorsque le matériau solide de stockage de l'ozone selon l'invention est sous forme compactée, il peut se présenter sous forme de granulés, d'une pastille et/ou d'une briquette.

Lorsque le matériau solide de stockage de l'ozone selon l'invention, compacté ou non, est sous forme conditionnée, il est disposé dans un contenant tel qu'un sachet, un tube, une boîte, un flacon, une colonne, une capsule ou une gélule. Ce contenant peut être éventuellement fermé hermétiquement. Ce contenant peut éventuellement être le réacteur dans lequel a eu lieu la mise en contact.

Que le matériau solide de stockage de l'ozone selon l'invention soit sous forme compactée et/ou sous forme conditionnée, il peut être conservé à une température comprise entre -80°C et 50°Cet notamment entre -80°C et 40°C et ce, sous vide, sous air ambiant, sous air humide, sous air sec, sous dioxyde de carbone, sous gaz inerte comme de l'argon, de l'azote ou un de leurs mélanges.

La présente invention concerne enfin l'utilisation d'un matériau solide de stockage de l'ozone tel que précédemment défini ou d'un matériau solide de stockage de l'ozone susceptible d'être préparé par un procédé de préparation tel que précédemment défini comme agent désinfectant (notamment éliminant les virus), dépolluant, nettoyant ou biocide (notamment fongicide, bactéricide ou herbicide). L'activité du matériau solide selon l'invention en tant qu'agent désinfectant, dépolluant, nettoyant ou biocide est générée par l'ozone stocké, sous forme physique et/ou chimique, dans ce matériau.

En d'autres termes, la présente invention concerne un procédé pour désinfecter, dépolluer ou nettoyer un fluide ou une surface, consistant à mettre en contact ce fluide ou cette surface avec un matériau solide de stockage de l'ozone tel que précédemment défini ou un matériau solide de stockage de l'ozone susceptible d'être préparé par un procédé de préparation tel que précédemment défini.

Par « désinfecter, dépolluer ou nettoyer un fluide ou une surface », on entend dans le cadre de la présente invention diminuer la quantité ou l'activité d'agents biologiques ou composés chimiques présents dans le fluide ou sur la surface avant la mise en contact avec le matériau solide de stockage de l'ozone selon l'invention. Cette diminution de quantité peut impliquer l'élimination ou la destruction de ces agents ou composés et/ou leur transformation en éléments moins nocifs.

Tout fluide susceptible d'être contaminé par un ou plusieurs agent(s) biologique(s) ou un ou plusieurs composé(s) chimique(s) peut être soumis à un procédé de désinfection, de dépollution ou de nettoyage selon la présente invention. Par « fluide », on entend aussi bien un gaz ou un liquide. Plus particulièrement, un tel fluide peut être choisi parmi l'air ambiant ou l'atmosphère gazeuse d'un site tel qu'une pièce domestique, une chambre froide ou un espace confiné industriel ; de l'eau de ville, de rivière, de puits, de nappe phréatique, d'étang, de lac, de piscine, d'un aquarium, de refroidissement des systèmes de climatisation ou de tours aéro-réfrigérées ; un prélèvement dans un réacteur chimique ; une eau usée domestique ; un produit notamment liquide, un effluent ou de l'eau usée provenant notamment d'élevages intensifs ou d'industries ou d'installations du domaine chimique, pharmaceutique, cosmétique, agricole, agroalimentaire, maritime, aéronautique ou spatial ; ou un de leurs mélanges. A noter que, lorsque le fluide est de l'air ambiant ou une atmosphère gazeuse, le matériau solide de stockage de l'ozone tel que précédemment défini ou le matériau solide de stockage de l'ozone susceptible d'être préparé par un procédé de préparation tel que précédemment défini permet de traiter ces derniers en le dépolluant, en le désinfectant et/ou en éliminant les odeurs.

Toute surface susceptible d'être contaminée par un ou plusieurs agent(s) biologique(s) ou un ou plusieurs composé(s) chimique(s) peut être soumise à un procédé de désinfection, de dépollution ou de nettoyage selon la présente invention. Avantageusement, dans le cadre de la présente invention, la surface à désinfecter, à dépolluer ou à nettoyer peut être une surface inorganique et notamment une surface en métal comme en aluminium, en alliage métallique, en acier et notamment en acier inoxydable, en fer-blanc, en silicium, en verre contenant généralement des silicates, en verre de silice, en céramique, en brique, en porcelaine, en ciment, en béton, en asphalte, en pierre, en granit, en plastique ou en une quelconque de leurs associations. Plus particulièrement, une telle surface peut être choisie parmi des installations de grande taille comme un objet industriel comme un appareil électronique ou une machine utilisée dans l'agroalimentaire, un véhicule, une carcasse, un aéronef, une cuve, une cuisine de restaurant, une chambre froide, un sanitaire, un conteneur, une partie d'une habitation comme un toit, une façade, une terrasse, une allée ; et des installations de petite taille comme des système embarqués dans l'espace, dans des bateaux ou dans des sous-marins, des dispositifs médicaux ou des tuyaux. Il peut également s'agir d'une surface organique comme un sol ou de la terre, du bois ou une surface végétale. Par « surface végétale », on entend une plante, une partie de plante comme les feuilles, les tiges, les racines, les fruits ou les graines, ou un ensemble de plantes.

Par « agent biologique », on entend aussi bien les micro-organismes naturels tels que bactéries, archées, parasites, protozoaires, champignons, levures ou virus, les toxines produites ou non par de tels micro-organismes, les agents pathogènes de nature protéique comme les prions et les micro-organismes génétiquement modifiés que les végétaux comme les plantes ou les mousses ou les parties de végétaux comme les graines, les fruits, les feuilles, les tiges ou les racines.

Eu égard à ce qui précède, il est clair que des végétaux ou parties de végétaux peuvent être, dans certaines applications, la surface à traiter. Dans ce cas, le matériau solide de stockage de l'ozone tel que précédemment défini ou le matériau solide de stockage de l'ozone susceptible d'être préparé par un procédé de préparation tel que précédemment défini sert à éliminer les micro-organismes comme les champignons ou les bactéries présents sur ces végétaux ou parties de végétaux. Dans d'autres applications, les végétaux ou parties de végétaux constituent l'agent biologique à éliminer et le matériau solide de stockage de l'ozone tel que précédemment défini ou le matériau solide de stockage de l'ozone susceptible d'être préparé par un procédé de préparation tel que précédemment défini devient un agent herbicide. L'homme du métier saura déterminer sans effort inventif et au besoin à l'aide de tests de routine, la quantité de matériau à utiliser en fonction de l'application visée.

Par « composé chimique », on entend un composé non souhaité comme un polluant ou un contaminant susceptible d'être présent ou présent dans un fluide ou sur une surface. A titre d'exemples illustratifs et non limitatifs, le composé peut être choisi parmi le dioxyde d'azote (NO₂), le monoxyde de carbone (CO), le dioxyde de soufre (SO₂), l'acroléine, un phénol, un insecticide, un pesticide, un composé soufré tel que du sulfure d'hydrogène (H₂S), un thiol ou un mercaptan, un hydrocarbure saturé ou insaturé tel qu'un alcène ou un hydrocarbure aromatique polycyclique, un composé organique volatil tel que un adhéhyde, du formaldéhyde, de l'acétaldéhyde, du naphtalène, une amine primaire notamment aromatique, de l'indole, du scatole, du tryptophane, de l'urobilinogène, du pyrrole, du benzène, l'éthylbenzène, du toluène, un xylène, du styrène, du napthalène, un composé halogéné, une toxine, un glucide, un peptide, une protéine, une glycoprotéine, un composé pharmaceutique, un dérivé pharmaceutique ou un de leurs mélanges.

La mise en contact entre le fluide ou la surface et le matériau solide de stockage de l'ozone selon l'invention peut être mise en œuvre de différentes façons et notamment en fonction de la nature gazeuse ou liquide du fluide. Ainsi, on peut introduire le matériau solide de stockage de l'ozone dans le fluide liquide, déposer ou appliquer le matériau solide de stockage de l'ozone sur la surface, mettre le matériau stockage de l'ozone en présence du fluide gazeux (expositions statiques) ou faire circuler le fluide notamment gazeux sur le matériau solide de stockage de l'ozone (exposition dynamique).

Dans certaines de ces variantes, il peut être avantageux de conditionner le matériau solide de stockage de l'ozone selon l'invention notamment sous forme de colonne dans laquelle le matériau selon l'invention correspond à un lit fluidisé dont le fluide liquide ou gazeux assure la fluidisation.

Dans le cas où le matériau solide de stockage de l'ozone selon l'invention est appliqué sur une surface, cette application peut se faire par saupoudrage du matériau solide ou par pulvérisation d'une solution, d'une dispersion, d'une émulsion, d'une micro-émulsion ou d'une suspension contenant le matériau.

Dans le cas où le matériau est introduit dans le fluide liquide, il peut être avantageux d'agiter le mélange ainsi obtenu.

Dans un mode de réalisation particulier, le procédé pour désinfecter, dépolluer ou nettoyer un fluide ou une surface selon l'invention, comprend les étapes consistant à :
- préparer un matériau solide de stockage de l'ozone selon le procédé de préparation tel que précédemment défini ;
- éventuellement récupérer le matériau solide de stockage de l'ozone ainsi préparé puis
- mettre en contact ce fluide ou cette surface avec un matériau solide de stockage de l'ozone ainsi préparé ou éventuellement ainsi récupéré.

Dans ce mode de réalisation particulier, il est possible de mettre en contact le matériau solide de stockage de l'ozone avec le fluide ou avec la surface tout de suite après sa préparation ou sa récupération. En variante, une fois préparé et avant la mise en contact, le matériau solide de stockage de l'ozone peut être conservé ou stocké. Typiquement, cette conservation ou stockage peut être réalisé(e) à une température comprise entre -80°C et 50°Cet notamment entre -80°C et 40°C et ce, sous vide, sous air ambiant, sous air humide, sous air sec, sous dioxyde de carbone ou sous gaz inerte comme de l'argon, de l'azote ou un de leurs mélanges.

La présente invention concerne aussi un matériau solide de stockage de l'ozone tel que précédemment défini ou un matériau solide de stockage de l'ozone susceptible d'être préparé par un procédé de préparation tel que précédemment défini pour utilisation comme médicament. En effet, du fait des propriétés biocides de ce matériau, on peut envisager de l'utiliser pour le traitement ou la prévention de pathologies ou troubles provoqués par un micro-organisme tel que précédemment défini. En particulier, ces pathologies ou ces troubles sont des pathologies ou troubles cutanés. A titre d'exemples illustratifs de telles pathologies ou de tels troubles, on peut citer un impétigo, une lymphangite, un furoncle, un abcès, un anthrax, une mycose, une verrue, un eczéma, une dermatite séborrhéique, un zona et un herpès. Par « médicament », on entend aussi bien un médicament à usage humain qu'un médicament à usage vétérinaire.

La présente invention concerne aussi l'utilisation d'un matériau solide de stockage de l'ozone tel que précédemment défini ou d'un matériau solide de stockage de l'ozone susceptible d'être préparé par un procédé de préparation tel que précédemment défini comme un réactif chimique. En effet, ce matériau peut être utilisé comme agent oxydant lors d'une réaction chimique et/ou pour fournir l'ozone durant une réaction chimique en nécessitant.

D'autres caractéristiques et avantages de la présente invention apparaîtront encore à l'homme du métier à la lecture des exemples ci-dessous donnés à titre illustratif et non limitatif, en référence aux figures annexées.

### Brève description des figures

La Figure la présente un schéma d'une installation susceptible d'être mise en œuvre dans le cadre de la présente invention avec 1. Ozoneur; 2. Réacteur; 3. Filtre; 4. Destructeur ; F1. Débitmètre ; T1, T2. Sondes de température ; A1. Analyseur d'ozone ; V1, V2, V3. Vannes.
La Figure 1b présente un schéma d'une installation susceptible d'être mise en œuvre dans le cadre de la présente invention avec 1. Ozoneur ; 2. Réacteur ; 3. Bain thermostaté ; 4. Pompe à vide ; 5. Filtre ; 6. Destructeur; F1, F2. Débitmètres ; T1. Sonde de température ; P1, P2. Capteurs de pression ; A1. Analyseur d'ozone ; V1, V2, V3, V4, V5, V6, V7, V8, V9, V10, V11. Vannes.
La Figure 2 présente les résultats d'un test à l'iodure de potassium (KI) réalisé avec de la HP-β-CD native avant réaction (fiole du milieu = test négatif) et après réaction (fiole de droite = test positif), la fiole de gauche étant un contrôle négatif ne contenant que du KI. Le test a été réalisé juste après la synthèse (à J₀).
La Figure 3 présente les résultats des tests biologiques dans les conditions détaillées dans le Tableau 1.
La Figure 4 présente les résultats des tests biologiques dans les conditions détaillées dans le Tableau 2.
La Figure 5 présente les résultats d'un test à l'iodure de potassium (KI) en fonction du temps de stockage de la β-CD après réaction. De gauche à droite : KI seul (contrôle négatif), KI + β-CD native avant réaction, KI + β-CD après réaction à t₀, KI + β-CD après réaction à t₀ + 1 jour, KI + β-CD après réaction à t₀ + 2 jours, KI + β-CD après réaction à t₀ + 5 jours et KI + β-CD après réaction à to + 6 jours.
La Figure 6 présente l'évolution de la concentration massique d'ozone contenue dans le matériau au cours du temps pour 3 températures de stockage testées (-19°C, 2°C et 21°C).

### Exposé détaillé des modes de réalisation particuliers

### I. Installation et procédé de préparation du matériau selon l'invention.

### I.1. Exemple 1 d'installation et de procédé de préparation du matériau selon l'invention.

Un exemple particulier d'installation mise en œuvre pour préparer un matériau à activité oxydative selon l'invention est décrit Figure la.

Cette installation se compose d'un ozoneur (1), un réacteur (2), un filtre (3) et un destructeur d'ozone (4). Les 3 vannes (V1), (V2), (V3) permettent de diriger ou non le gaz vers le réacteur. Les paramètres du procédé sont suivis à l'aide de différents capteurs qui sont, pour certains, reliés à des afficheurs : un débitmètre gaz (rotamètre à bille (F1)) placé en sortie d'ozoneur, une sonde de température (T1) en entrée du réacteur, une sonde de température (T2) en sortie du réacteur, et un analyseur d'ozone (A1) placé entre le filtre et le destructeur.

La synthèse du matériau d'intérêt se fait par réaction directe gaz/solide entre les cyclodextrines et un mélange gazeux dioxygène/ozone (O₂/O₃). Les cyclodextrines utilisées dans la partie expérimentale ci-après sont la β-cyclodextrine (β-CD, fournie par Sigma-Aldrich, fabriquée par Wacker Chemie AG, Burghausen, Germany, Life Science, Iot BCBG7824V, pureté de 98,6%) et la (2-hydroxypropyl)-β-cyclodextrine (HP-β-CD, fournie par Sigma-Aldrich, fabriquée par Wacker Chemie AG, Burghausen, Germany, Life Science, Iot BCBV0722, pureté supérieure à 94%) qui est beaucoup plus soluble dans l'eau que la β-CD. Ces cyclodextrines se présentent sous forme de poudre de granulométrie fine avec un diamètre moyen des grains inférieur à 100 µm et, plus particulièrement, de 60 µm pour β-CD et de 13 µm pour HP-β-CD.

Le réacteur (2) utilisé est composé d'un tube de verre de 20 cm de hauteur, de 6 mm de diamètre externe et d'1 mm d'épaisseur. Le tube est serti aux extrémités par des raccords « double bague » en PTFE. Le tube verre muni des raccords est fixé sur des supports en inox, en position verticale, avec une alimentation en gaz en partie basse. Ce réacteur est positionné dans un four (Heratherm oven OGS60) pour faire varier, le cas échéant, la température.

Le matériau avant réaction, par exemple, des cyclodextrines natives, est initialement chargé dans le réacteur (2) manuellement. Le solide sous forme de poudre est maintenu dans le réacteur via deux filtres positionnés en amont et en aval de la poudre. Les deux filtres utilisés sont en coton dans les expériences réalisées et sont positionnés de telle façon que la poudre puisse être fluidisée par le gaz alimentant le réacteur.

Le filtre (3) permet d'éviter l'entraînement de fines particules dans le destructeur.

Le destructeur d'ozone (4) est un destructeur thermo-catalytique de type COD 8 en acier inoxydable 316 Ti, capable de traiter instantanément un débit maximum de gaz de 8 Nm³/h. Le catalyseur est constitué par du dioxyde de manganèse et de l'oxyde de cuivre déposés sur de l'oxyde d'alumine. Au contact de cette masse catalytique, les molécules d'ozone sont décomposées sous forme de molécules de dioxygène avant d'être rejetées vers l'atmosphère (vent).

Pour la phase de démarrage, les vannes sont initialement positionnées de telle façon que le gaz sortant de l'ozoneur soit dirigé vers le destructeur d'ozone : vannes (V1) et (V3) fermées et (V2) ouverte.

L'ozone gazeux est généré par un ozoneur *Trailigaz de type Labo5LO* à décharge électrique, alimenté en dioxygène pur (O₂). La production d'ozone est modifiée régulée par le réglage de la tension sur le potentiomètre. Le gaz produit en sortie d'ozoneur est un mélange O₂/O₃. Le débit de ce gaz en sortie d'ozoneur est réglé et mesuré en utilisant le débitmètre (F1) et la concentration d'ozone est mesurée grâce à l'analyseur (A1).

La tension de l'ozoneur est augmentée jusqu'à atteindre la concentration d'ozone désirée dans le gaz. Cette concentration a été fixée entre 55 et 75 g/Nm³ pour les expériences. Lors de cette phase transitoire, le flux gazeux ne passe pas dans le réacteur.

Une fois que la concentration d'ozone sur l'analyseur (A1) et les températures (T1) et (T2) sont stables et conformes aux valeurs désirées (entre 23 et 26°C pour les expériences), le gaz sortant de l'ozoneur est dirigé vers le réacteur en fermant la vanne (V2) et en ouvrant les vannes (V1) et (V3). Dans les expériences réalisées, le débit de gaz a été fixé à 30 L/h avec le débitmètre (F1). Dans ces conditions, la vitesse de passage du gaz (0,66 m/s) est suffisante pour fluidiser le matériau présent dans le réacteur. Le temps de passage du mélange gazeux dans le réacteur est alors de 0,3 s.

La durée de mise en contact de la poudre avec le gaz contenant de l'ozone a été fixée arbitrairement à 3 heures pour ces expériences.

Lorsque le temps de réaction souhaité est atteint, les vannes sont positionnées de telle façon que le gaz sortant de l'ozoneur soit dirigé vers le destructeur. Le réacteur est alors démonté du support, les filtres en coton sont enlevés avec une pince et la poudre traitée à l'ozone est récupérée dans un flacon en verre.

Le matériau obtenu en fin de synthèse est une fine poudre blanche, ressemblant, à l'œil nu, au matériau préalablement au procédé selon l'invention.

Le produit est stocké sans précautions particulières dans des flacons fermés avec un bouchon. Deux conditions de stockage ont été testées : conditions dites « ambiantes » (pression atmosphérique et température d'environ 25°C) ou stockage à froid (pression atmosphérique et température d'environ 6°C).

### I.2. Exemple 2 d'installation et de procédé de préparation du matériau selon l'invention.

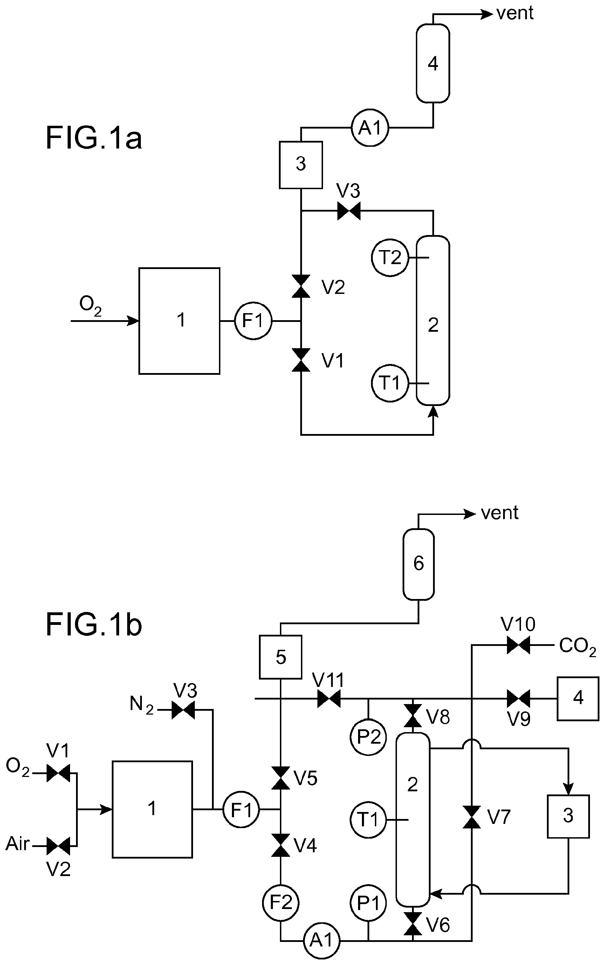

Un autre exemple particulier d'installation mise en œuvre pour préparer un matériau à activité oxydative selon l'invention est décrit Figure 1b.

Cette installation se compose d'un ozoneur (1), un réacteur (2), un bain thermostaté (3), une pompe à vide (4), un filtre (5) et un destructeur d'ozone (6). Les vannes (V1) et (V2) permettent de choisir le gaz d'alimentation de l'ozoneur (oxygène ou air). Les vannes (V3) et (V10) permettent de faire circuler de l'azote ou du CO₂ dans le procédé, si besoin. La vanne (V4) permet de diriger le gaz venant de l'ozoneur vers le réacteur (2). La vanne (V5) dirige le gaz vers le destructeur (5). Les vannes (V6) et (V8) permettent d'isoler le réacteur (2), et la vanne (V7) d'ouvrir le bypass du réacteur (2). La vanne (V9) permet de connecter la pompe à vide (4) au procédé, et la vanne (V11) permet de fermer le circuit de purge du réacteur (2) pour la mise sous vide de l'installation. Les paramètres du procédé sont suivis à l'aide de différents capteurs qui sont reliés, pour certains, à un système d'acquisition et à un ordinateur: un débitmètre volumique (rotamètre à bille (F1)) placé en sortie d'ozoneur permettant de régler les débits de gaz d'alimentation du réacteur (2), un débitmètre massique (F2) pour mesurer précisément le débit d'alimentation du réacteur (2), un analyseur d'ozone (A1) pour mesurer la concentration en ozone du gaz d'alimentation du réacteur (2), deux capteurs de pression (P1) et (P2) permettant de mesurer la pression en amont et en aval du réacteur (2), et une sonde de température (T1) permettant de mesurer la température au sein du réacteur (2).

La synthèse du matériau d'intérêt se fait par réaction directe gaz/solide entre les cyclodextrines (CDs) et un mélange gazeux contenant de l'ozone (O₃). Les cyclodextrines (CD) utilisées dans la partie expérimentale en lien avec cette installation sont l'a-cyclodextrine (α-CD, fournie par Sigma-Aldrich, fabriquée par Wacker Chemie AG, Burghausen, Germany, Life Science, Iot BCBQ5117V, pureté de 98), la β-cyclodextrine (β-CD, fournie par Sigma-Aldrich, fabriquée par Wacker Chemie AG, Burghausen, Germany, Life Science, Iot BCBG7824V, pureté de 98,6%), la γ-cyclodextrine (γ-CD, fournie par Sigma-Aldrich, fabriquée par Wacker Chemie AG, Burghausen, Germany, Life Science, Iot BCBG7825, pureté de 99,5 %), la (2-hydroxypropyl)-β-cyclodextrine (HP-β-CD, fournie par Sigma-Aldrich, fabriquée par Wacker Chemie AG, Burghausen, Germany, lots BCBV0722 et BCBX5180, pureté supérieure à 94%), la Sulfobutylether-β-Cyclodextrine (SBE-β-CD, fournie par ABMole, lots M4837-07, pureté égale à 98,08 %) et un polymère de cyclodextrine (β-CD polymer, fournie par Sigma-Aldrich, numéro de produit C2485, Iot BCBX7555).

Le réacteur (2) utilisé est entièrement un inox. C'est un réacteur tubulaire de 17 mm de diamètre, 150 mm de longueur totale et 50 mm de longueur utile, qui peut contenir jusqu'à environ 5 g de poudre de cyclodextrine. Il est muni d'une double enveloppe en inox où circule un fluide caloporteur circulant dans des tuyaux flexibles. Ces tuyaux sont connectés au bain thermostaté (3) qui assure la mise en température et la circulation du fluide caloporteur dans la double enveloppe du réacteur. Le réacteur est monté en position verticale, avec une alimentation en gaz en partie basse, et est connecté au reste du procédé par des raccords double bague en inox.

Le matériau avant réaction, constitué par des cyclodextrines natives, est initialement chargé manuellement dans le réacteur (2). Le solide sous forme de poudre est maintenu dans le réacteur via deux frittés positionnés en amont et en aval de la poudre.

La pompe à vide (4) permet de mettre le réacteur et une partie de l'installation sous vide, lorsque nécessaire.

Le filtre (5) permet d'éviter l'entraînement de fines particules dans le destructeur.

Le destructeur d'ozone (6) est un destructeur thermo-catalytique. Au contact de du catalyseur et sous l'effet de la température, les molécules d'ozone sont décomposées sous forme de molécules de dioxygène avant d'être rejetées dans l'atmosphère (vent).

Pour la phase de démarrage, les vannes sont initialement positionnées de telle façon que le gaz sortant de l'ozoneur soit dirigé vers le destructeur d'ozone en passant par le bypass du réacteur: vannes (V5), (V6) et (V8) fermées, et vanne (V7) ouverte.

L'ozone gazeux est généré par un ozoneur (1) (modèle CFS-01-2G de chez Ozonia). Il utilise un procédé de décharge diélectrique à partir d'air sec ou d'oxygène. La production d'ozone est réglée directement sur l'ozoneur par une valeur de puissance. Le gaz produit en sortie d'ozoneur est soit un mélange O₂/O₃ lorsque de l'oxygène pur est utilisé comme gaz d'alimentation de l'ozoneur, soit un mélange N₂/O₂/O₃ si de l'air est utilisé à la place de l'oxygène pur. Le débit de ce gaz en sortie d'ozoneur est réglé et mesuré en utilisant d'abord le rotamètre (F1) puis le débitmètre (F2). La concentration d'ozone est mesurée grâce à l'analyseur (A1).

La puissance de l'ozoneur est augmentée jusqu'à atteindre la concentration d'ozone désirée dans le gaz. Lors de cette phase transitoire, le flux gazeux ne passe pas dans le réacteur. Lors des expériences réalisées avec cette installation, la concentration d'ozone peut être très élevée, par exemple égale à 165 g O₃/Nm³.

La température dans le réacteur est régulée par le bain thermostaté (3). La plage de température des expériences réalisées avec cette installation se situe entre 7 et 77°C.

Une fois que la concentration d'ozone sur l'analyseur (A1) et la température (T1) sont stables et conformes aux valeurs désirées, le gaz sortant de l'ozoneur est dirigé vers le réacteur en fermant la vanne (V7) et en ouvrant les vannes (V6) et (V8). Dans les expériences réalisées, le débit de gaz peut être variable, et a varié de 33 à 723 Normaux litres par heure (NI/h) avec les débitmètres (F1) et (F2). La durée de mise en contact de la poudre avec le gaz contenant de l'ozone peut également être variable, pouvant aller de 0,5 à 6 h pour ces expériences.

Lorsque le temps de réaction souhaité est atteint, les vannes sont positionnées de telle façon que le gaz sortant de l'ozoneur soit dirigé vers le destructeur. Le réacteur est alors démonté du support, les frittés sont enlevés.

Le matériau obtenu en fin de synthèse est une fine poudre, ressemblant, à l'œil nu, au matériau préalablement au procédé selon l'invention.

La poudre traitée à l'ozone est alors récupérée et stockée dans un flacon en verre, ou mise en forme (sous forme de pastille par exemple).

### II. Caractérisation du matériau selon la présente invention.

### II.1. Caractérisations et dosages.

Des tests de caractérisation (analyse thermogravimétrique couplée à une analyse calorimétrique différentielle, spectroscopie Infrarouge...) et des dosages (dosage à l'iodure de potassium/thiosulfate de sodium - méthode de dosage appelée « méthode au KI » ou « test au KI » - permettant de déterminer la quantité d'ozone contenue dans la poudre) ont été effectués sur certains des matériaux.

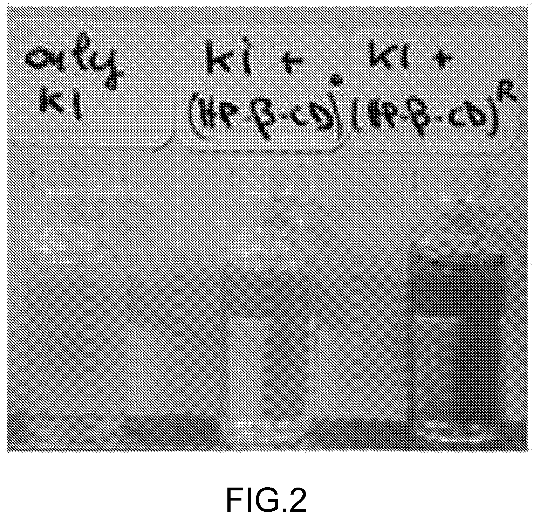

A noter que la dissolution de cyclodextrines natives (produit commercial) dans une solution de KI ne produit aucune coloration de la solution (test KI négatif). Uniquement les cyclodextrines ayant réagi à l'ozone obtenues selon le procédé de l'invention ont un test au KI dit « positif » : la solution devient jaune/orange comme illustré sur la Figure 2.

### 11.2. Protocole détaillé des tests biologiques.

Les tests microbiologiques ont pour objectif de vérifier l'effet biocide du matériau selon la présente invention et ainsi d'évaluer son potentiel pour un usage dans la protection des cultures par exemple.

### II.2.1. Protocole A

Des supports artificiels sont tout d'abord placés dans des boîtes de Pétri (2 boîtes/modalité testées) sans milieu gélosé puis sont inoculés à l'aide d'une solution de micro-organismes (champignons ou bactéries), par exemple conidies de *Venturia inaequalis,* champignon responsable de la tavelure du pommier. Ces supports sont ensuite placés 24 h dans une enceinte d'incubation afin d'initier le développement des micro-organismes.

Les traitements avec un matériau selon la présente invention sont appliqués par saupoudrage (0,1 g/boîte) 24 h après le début de la germination.

Après une heure environ de mise en contact entre les micro-organismes et le matériau, les supports artificiels sont déplacés sur milieu gélosé (Patato Dextrose Agar) afin d'assurer l'apport nutritif nécessaire au bon développement des micro-organismes. Les boîtes de Pétri inoculées traitées sont ensuite placées dans une enceinte climatisée (cycle jour/nuit 12h/12h, température nuit : 8°C, température jour : 17°C).

A partir du 3^{ième} jour d'incubation, des observations et comptages réguliers sont réalisés afin d'évaluer et comparer les différentes modalités.

### II.2.2. Protocole B

### Souches fongiques :

Trois souches fongiques ont été testées. Les souches *110.712* et *100.398* appartiennent à l'espèce *Pheaoacremonium minimum.* La souche 239.74 correspond, quant à elle, à l'espèce *Phaeomoniella chlamydospora.* Elles sont respectivement nommées *P.min 110.712, P.min 100.398 et P.ch* 239.74 ci-après. Elles sont toutes trois associées aux maladies du bois de la vigne connues sous le nom d'Esca.

Elles ont, tout d'abord, été cultivées pendant quatre semaines sur milieu gélosé (Malt Extract Agar ou MEA) afin de leur permettre d'atteindre le stade de sporulation. Le jour du test, une suspension de spores d'une concentration d'environ 1.10⁵ spores/mL a été préparée pour chacune des souches. Le comptage a été opéré sur cellules de Malassez. Les suspensions de spores ainsi obtenues sont réparties dans 8 eppendorfs (1,5 mL de suspension par eppendorf).

La mise en contact de la poudre native ou ozonée et des suspensions de spores est de 20 min dans la glace. Les échantillons sont ensuite dilués et ensemencés sur milieu gélosé (MEA). Les boîtes de Pétri sont incubés pendant 5 jours à l'obscurité à 26°C. Les comptages sont effectués après ce temps d'incubation et permettront de comparer les échantillons ayant reçu de la poudre native sans ozone *versus* ceux ayant reçu de la poudre ozonée.

### Souches bactériennes :

Deux espèces ont été testées : *Escherichia coli* et *Streptococcus uberis (E.coli* et *S.uberis).* Les bactéries, qui étaient jusqu'alors stockées dans du lait glycérolé à -80°C, ont été déposées dans un milieu BCC (Bouillon Cœur Cervelle) liquide, préalablement autoclavé 15 min à 121°C. Dans le but de les sortir de la phase de latence et de leur permettre d'atteindre la phase exponentielle de croissance, les bactéries ont subi une préculture respectivement de 4h et 4h30 à 37°C sous une agitation de 150 rpm.

Dès la mise en contact avec le matériau natif ou le matériau ozonée, 150 µL de suspension de spores ont été déposés sur des plaques 96 puits afin de lire l'absorbance toutes les 10 min pendant 4 h à 600 nm par le biais du TECAN (agitation 150 rpm, 37°C). Les différentes modalités sont ensuite comparées.

### II.3. Résultats.

Trois séries de tests (Campagnes de tests N°1, N°2 et N°3) ont été effectuées (3 h de réaction à température ambiante (Tₐₘ) environ 25°C, avec des produits natifs utilisés sans aucun traitement préalable) afin d'évaluer : (i) l'effet du type de cyclodextrines (β-CD et HP-β-CD) ; (ii) l'influence des conditions de stockage du matériau après réaction (Tₐₘ ou 6°C) ; (iii) la quantité d'ozone contenue dans le matériau ; (iv) la reproductibilité ; (iv) l'efficacité du matériau (tests biologiques selon le protocole A (point 11.2.1. ci-dessus).

Des tests complémentaires (Campagne de tests N°4), ont été effectuées pour s'assurer que les capacités désinfectantes des β-CD oxydantes i.e. préparées selon le procédé de l'invention sont vérifiées sur plusieurs souches bactériennes et fongiques. Ces essais ont été réalisés selon le protocole B (point II.2.2. ci-dessus) avec des CD oxydantes obtenues à partir de l'HP-β-CD selon l'exemple 2 de procédé (point I.2. ci-dessus).

### 11.3.1. Campagne de tests N°1 : β-CD

Les synthèses ont été réalisées avec la β-CD selon l'exemple 1 de procédé (point I.1. ci-dessus). Le Iot de poudre en fin de synthèse a été divisé en deux : une partie stockée en conditions ambiantes et une à 6°C. Les caractérisations analytiques du produit formé ont été faites 48 h après la fin de synthèse (J₀+2) au moment des tests biologiques (Tests Bio). Les résultats sont résumés dans le Tableau 1 ci-après.

**Tableau 1 : Résumé des tests N°1**

| | | | | | TESTS BIO(^{∗}) | | |
|---|---|---|---|---|---|---|---|
| exp | ech | T stockage | Test KI | [O₃] µg/g_{poudre} | m_{poudre} Test bio | Pathogènes | Efficacité biologique |
| 1 | 1 | Tₐₘ | - | | 0,1 g | Bactéries + champignons | Partielle (croissance ralentie) |
| 1 | 2 | 6°C | - | | 0,1 g | Bactéries + champignons | |
| 2 | 3 | Tₐₘ | Positif | 725(^{∗}) | 0,1 g | Bactéries + champignons | |
| 2 | 4 | 6°C | Positif | 817(^{∗}) | 0,1 g | Bactéries + champignons | |

| | | | | | | | |
|---|---|---|---|---|---|---|---|
| (^{∗}) fait à I₀+2 | | | | | | | |

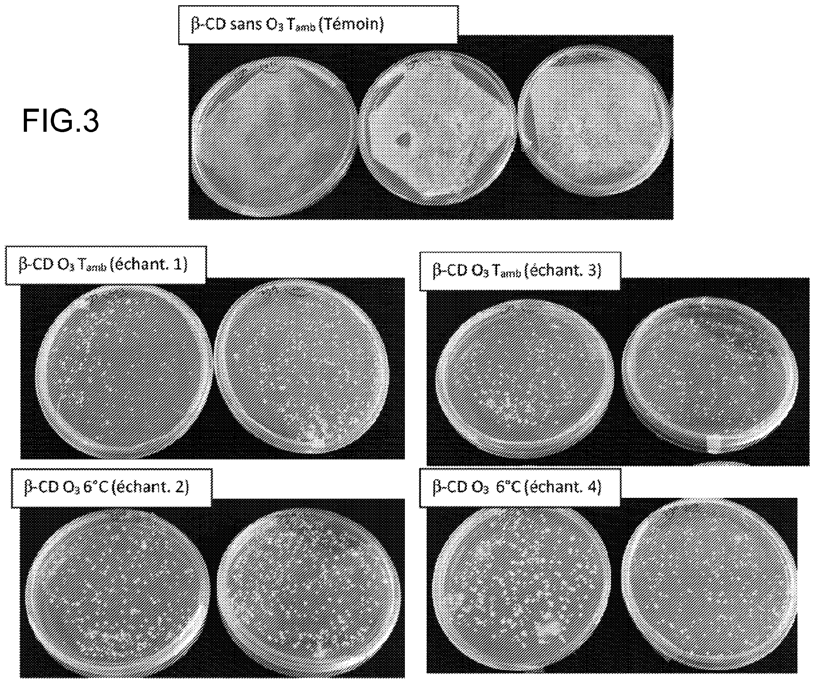

Les résultats des tests biologiques sont également présentés Figure 3. Les boîtes Témoins présentent un nombre non dénombrable de micro-organismes (aspect de tapis). En revanche, les boîtes ayant reçu des β-CD oxydantes i.e. préparées selon le procédé de l'invention sont, quant à elles, largement plus clairsemées. L'effet est donc avéré même s'il n'est ici que partiel car tous les micro-organismes n'ont pu être contraints.

### 11.3.2. Campagne de tests N°2 : MP-β-CD

Les synthèses ont été réalisées avec la HP-β-CD selon l'exemple 1 de procédé (point I.1. ci-dessus). Le Iot de poudre en fin de synthèse a été divisé en deux : une partie stockée en conditions ambiantes (environ 25°C) et une à 6°C. Les caractérisations analytiques du produit formé ont été faites 24 h après la fin de synthèse (J₀+1) au moment des tests biologiques (Tests Bio). Les résultats sont résumés dans le Tableau 2 ci-après.

**Tableau 2 : Résumé des tests N°2**

| | | | | | TESTS BIO(^{∗}) | | |
|---|---|---|---|---|---|---|---|
| exp | ech | T stockage | Test KI | [O₃] µg/g_{poudre} | m_{poudre} Test bio | Pathogènes | Efficacité biologique |
| 3 | 5 | Tₐₘ | - | | 0,1 g | Bactéries + champignons | Totale |
| 3 | 6 | 6°C | - | | 0,1 g | Bactéries + champignons | Totale |
| 4 | 7 | 6°C | Positif | 5716(^{∗}) | 0,1 g | Bactéries + champignons | Totale |

| | | | | | | | |
|---|---|---|---|---|---|---|---|
| (^{∗}) fait à J₀+1 | | | | | | | |

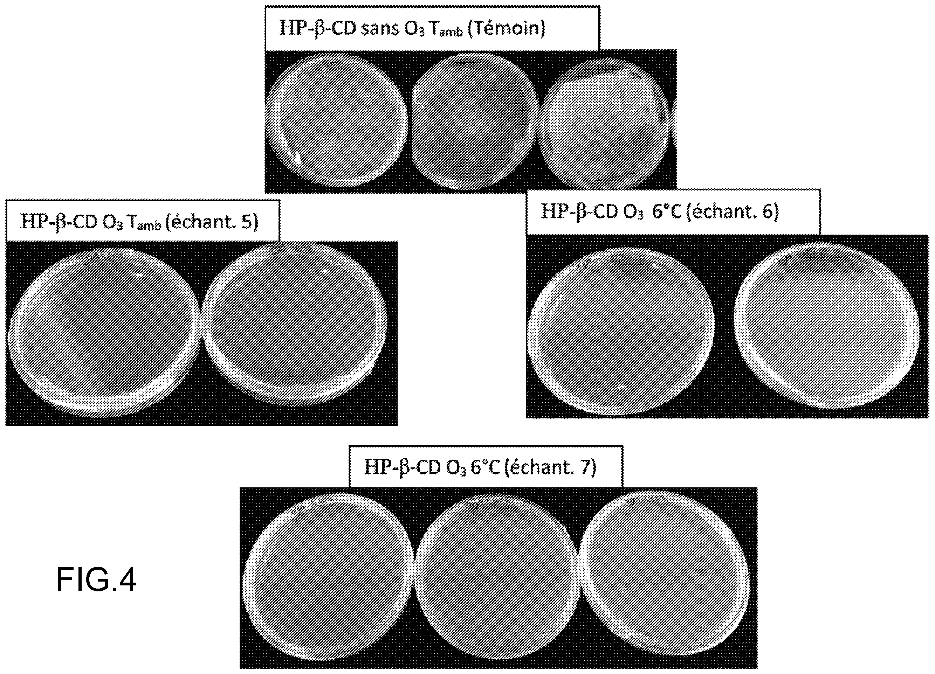

Les résultats des tests biologiques sont également présentés Figure 4. Les boîtes Témoins présentent à nouveau un nombre indénombrable de micro-organismes. En revanche, aucun micro-organisme à ce stade i.e. à J₀+1 n'est visible sur les boîtes ayant été traitées avec des HP-β-CD oxydantes i.e. préparées selon le procédé de l'invention.

### II.3.3. Campagne de tests N°3 : MP-β-CD

Les synthèses ont été réalisées avec la HP-β-CD selon l'exemple 1 de procédé (point I.1. ci-dessus). Le Iot de poudre en fin de synthèse a été conservé en totalité en conditions ambiantes. Le dosage d'ozone dans le matériau a été réalisé immédiatement après la synthèse (J₀), 24 h après la synthèse (J₀+1) et 48 h après la synthèse (J₀+2). Les résultats sont résumés dans le Tableau 3 ci-après.

**Tableau 3 : Résumé des tests N°3**

| | | | | | TESTS BIO | | |
|---|---|---|---|---|---|---|---|
| exp | ech | Date | Test KI | [O₃] µg/g_{poudre} | m_{poudre} Test bio | Pathogènes | Efficaci té biologique |
| 5 | 8_0 | J₀ | Positif | 5700 +- 100(^{∗}) | 0,05 g | champignons | Totale |
| | 8_1 | J₀+1 | Positif | 4713(^{∗∗}) | - | - | - |
| | 8_2 | J₀+2 | Positif | 3743(^{∗∗}) | - | - | - |

| | | | | | | | |
|---|---|---|---|---|---|---|---|
| (^{∗}) valeur moyenne et incertitude obtenue avec 3 dosages | | | | | | | |

Les valeurs indiquées (^{∗∗}) sont celles obtenues sur la première décoloration de la solution. On remarque que la solution se recolore en jaune progressivement après la fin du dosage. La valeur indiquée est donc probablement sous-estimée si la recoloration est due à une libération progressive d'ozone stabilisée dans la phase liquide. Des structures organisées présentes dans la solution sont clairement visibles au microscope : il est possible que les cyclodextrines en solution stabilisent un peu d'ozone, qui, de fait, ne réagit pas immédiatement durant le dosage.

### 11.3.4. Campagne de tests N°4

### Synthèse des matériaux selon l'invention :

Les synthèses ont été réalisées avec l'a-cyclodextrine (a-CD), la β-cyclodextrine (β-CD), la γ-cyclodextrine (y-CD), la (2-hydroxypropyl)-β-cyclodextrine (HP-β-CD), la Sulfobutylether-β-Cyclodextrine (SBE-β-CD) et un polymère de cyclodextrine (β-CD polymer) selon l'exemple 2 de procédé (point I.2. ci-dessus).

Les conditions opératoires de ces tests sont résumées dans le Tableau 4 ci-dessous. Les notations sont : Tr (température de réacteur) ; [O3]_{g} alim : concentration d'ozone dans le gaz d'alimentation ; Q = débit de gaz ; tₛ = temps de synthèse ; m = masse de poudre introduite dans le réacteur; [O3]ₚ = quantité d'ozone stockée obtenue par dosage volumétrique au KI ; Prétraitement: prétraitement de la poudre avant réaction; Alim ozoneur = nature du gaz d'alimentation de l'ozoneur.

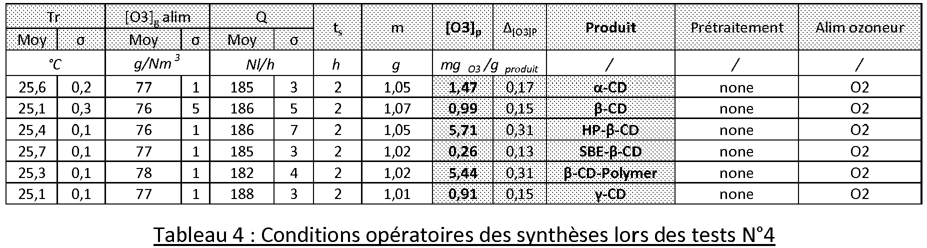

A conditions opératoire identiques, on note que la capacité de stockage d'ozone est fortement dépendante de la nature de la CD utilisée pour la synthèse, les meilleurs résultats étant obtenus avec l'HP-β-CD et le polymère de β-CD.

### Evaluation du caractère biocide d'un matériau ainsi préparé sur plusieurs souches bactériennes et fongiques

Les résultats sont résumés dans le Tableau 5 ci-après.

**Tableau 5 : Caractère biocide du matériau selon l'invention**

| Pathogènes | | Efficacité biologique |
|---|---|---|
| *Champignons* | *P.min 110.712* | Avérée |
| | *P.min 100.398* | *(spores dans l'incapacité de se développer après 5 jours d'incubation)* |
| | *P.ch* 239.74 | |
| Bactéries | *E.coli* | Avérée |
| | *S.uberis* | *(Arrêt de la croissance)* |

Concernant les souches fongiques, les boîtes Témoins présentent un nombre non dénombrable de micro-organismes (aspect de tapis). En revanche, les boîtes ayant reçu des β-CD oxydantes i.e. préparées selon le procédé de l'invention sont, quant à elles, largement plus clairsemées ou totalement exemptes de spots mycéliens. A la dilution au 100^{ième}, les milieux gélosés témoins comptent encore de nombreux départs mycéliens (en moyenne 990/ml déposé) alors que les milieux gélosés ayant reçu des β-CD oxydantes i.e. préparées selon le procédé de l'invention n'en comptent plus aucun pour les souches *P.min 100.398* et *P.ch.* 239.74. Seuls quelques départs mycéliens sont visibles pour la souche *P.min 110.712* qui semble un peu moins sensible. L'effet fongicide est donc confirmé.

En ce qui concerne les souches bactériennes, l'addition de β-CD oxydantes i.e. préparées selon le procédé de l'invention stoppe dans les deux cas le développement des bactéries alors que les témoins continuent leur croissance pendant les heures d'analyse. Là-encore, il est possible de conclure que le matériau nouvellement obtenu à un effet bactéricide.

### 11.3.5. Stabilité du matériau selon la présente invention.

Afin de tester la stabilité du matériau selon la présente invention, une synthèse a été réalisée avec la β-CD et un temps de mise en contact avec l'ozone de 2 h (selon l'exemple 1 de procédé du point I.1. ci-dessus). Le Iot de poudre en fin de synthèse a été stocké à 6°C (flacon ouvert).

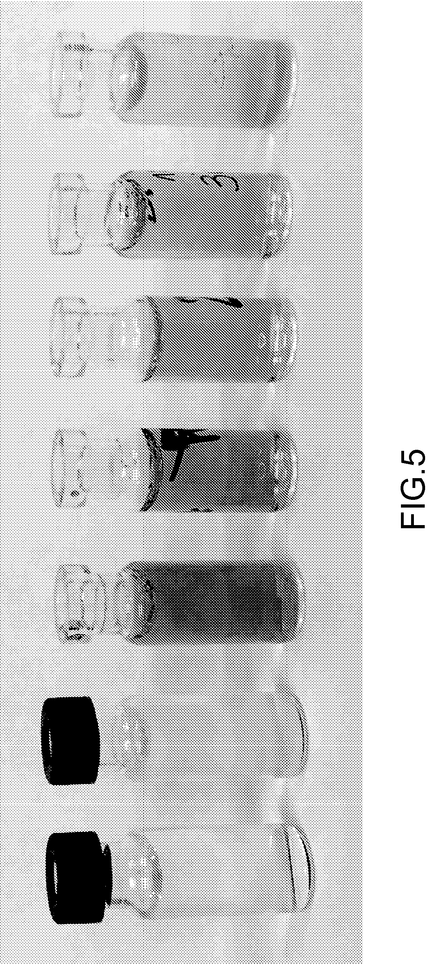

La Figure 5 présente une série de tests au KI effectués sur la poudre à différents temps de stockage (1 jour, 2 jours, 5 jours et 6 jours). On remarque une couleur jaune obtenue pour tous les échantillons contenant de la β-CD soumise au procédé selon l'invention, à comparer avec la couleur transparente du KI seul (fiole de gauche) ou du KI avec la β-CD avant la réaction (deuxième fiole en partant de la gauche).

Ce test de stabilité a été renouvelé avec la HP-β-CD traitée à l'ozone selon l'exemple 2 de procédé (point I.2. ci-dessous) dans les conditions suivantes : temps de synthèse = 6 h ; concentration d'ozone dans le gaz d'alimentation = 69 ± 18 g_{O3}/Nm³ ; débit de gaz = 335 ± 7 NI/h ; température de réacteur = 27,1°C ± 0,5°C. Le matériau a été conditionné dans des flacons en verre fermés. La stabilité du matériau a été évaluée sur une période de 65 jours dans différentes conditions de température : en conditions ambiantes à une température moyenne de 21°C ± 2 °C ; dans un réfrigérateur à une température moyenne de 2°C ± 2°C, et dans un congélateur à une température moyenne de -19°C ± 2 °C. La concentration d'ozone dans le matériau a été évaluée par dosage volumétrique (« méthode au KI »).

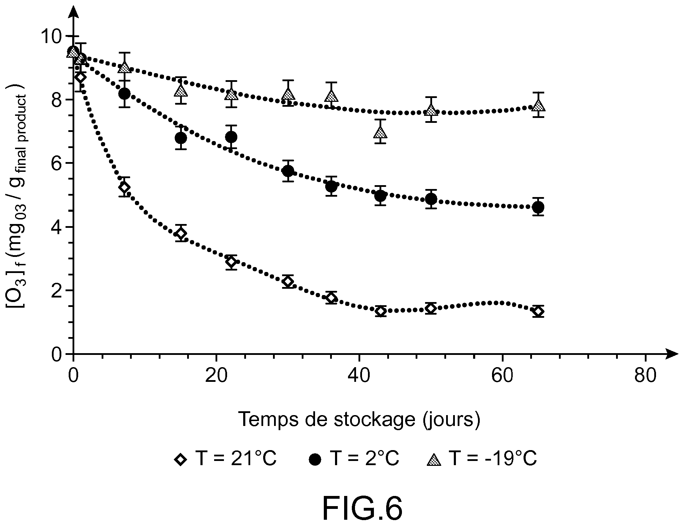

Les résultats quant à l'évolution de la concentration massique d'ozone contenue dans le matériau au cours du temps pour les 3 températures de stockage testées (-19°C, 2°C et 21°C) sont présentés Figure 6.

On peut donc conclure que le matériau préparé selon la présente invention stabilise l'ozone, ce qui permet de conserver ses propriétés oxydatives au minimum pendant plusieurs semaines. Il est à noter que le matériau est d'autant plus stable que la température de stockage est basse. A titre d'exemple, le taux de perte massique d'ozone (calculé comme 100 x [1 - (C/C₀)], avec C la concentration massique d'ozone au temps t et C₀ la concentration massique d'ozone initiale) est inférieur à 20% au bout de 65 jours de stockage si le matériau est maintenu à une température de -19°C.

Lors de tests complémentaires réalisés avec de l'HP-β-CD stockée à température ambiante durant 33 jours, il a également été montré que la stabilité du matériau au cours du temps n'était affectée ni par un conditionnement sous vide primaire, ni par un conditionnement sous 3,5 ± 0,2 bars absolus de CO₂.

### 11.3.6. Stockage de l'ozone par le matériau selon la présente invention.

Afin de valider le stockage d'ozone dans le matériau selon l'invention, deux tests ont été réalisés avec de l'HP-β-CD traitée selon l'exemple 2 de procédé (point I.2. ci-dessus).

### Premier test :

Lors du premier test, environ 1,5 g de matériau de stockage d'ozone sous forme de poudre a été placé dans une coupelle en verre, elle-même placée dans un réacteur en verre de volume 1,4 litre. Le réacteur peut être fermé hermétiquement et a été maintenu à température ambiante (~ 20°C).

Un détecteur portatif d'ozone (modèle X-an-5000 de chez Dräger, équipé d'une cellule XXS O₃ spécifique pour la détection d'ozone, limite de détection égale à 0,02 ppm et résolution égale à 0,01 ppm, temps de réponse < 10 s à 20°C) a été introduit dans le réacteur et allumé à proximité de la coupelle de poudre. Le détecteur ainsi placé permet de mettre en évidence en continu la présence d'ozone dans le réacteur, à partir d'une concentration d'ozone supérieure à la limite de détection de 0,02 ppm.

Dans un premier temps, il a été noté que le détecteur indique zéro ppm d'ozone lorsque la poudre est laissée telle quelle (i.e. le détecteur est allumé à côté de la poudre pendant plusieurs minutes et ne détecte pas d'ozone).

Dans un second temps, à l'aide d'une pipette en verre graduée, un volume d'environ 3 ml d'eau distillée a été introduit sur la poudre par l'intermédiaire d'un des piquages supérieurs du réacteur. Le réacteur a ensuite été refermé immédiatement et hermétiquement pour suivre l'évolution de la composition du ciel gazeux du réacteur avec le détecteur au cours du temps. La dissolution d'une partie du matériau (environ 1/3 de la quantité initiale) par cet ajout d'eau (car le matériau est relativement soluble dans l'eau), a provoqué une rapide augmentation de la concentration d'ozone au cours du temps lue sur le détecteur (exemple : 0,1 ppm mesuré en 40 s ; 0,2 ppm en 100 s). La concentration maximale atteinte a été de 0,48 ppm d'ozone pour cette expérience (concentration bien au-delà de la limite de détection de l'appareil de mesure).

Lors d'une autre expérience réalisée dans exactement les mêmes conditions, aucun dégagement d'ozone n'a été observé sur le détecteur lorsque du produit natif (c'est-à-dire HP-β-CD non ozonée) a été utilisé. Ces expériences confirment donc que le matériau fabriqué selon la présente invention stocke bien de l'ozone, et que de l'ozone sous forme gazeuse est libérée lorsque le matériau est mis en contact avec de l'eau.

### Second test :

Lors du second, environ 2 g de matériau de stockage d'ozone sous forme de poudre ont été placés dans une coupelle en verre, elle-même placée dans un réacteur en verre de volume 1,7 litre. Le réacteur fermé hermétiquement est placé dans une étuve initialement à température ambiante.

Un détecteur portatif d'ozone (modèle Micro IV de chez GIG, calibré spécifiquement pour l'ozone, limite de détection égale à 1 ppm et résolution égale à 0,01 ppm, temps de réponse < 60 s) a été introduit dans le réacteur et allumé à proximité de la coupelle de poudre. Le détecteur ainsi placé permet de mettre en évidence en continu la présence d'ozone dans le réacteur, à partir d'une concentration d'ozone supérieure à la limite de détection de 0,01 ppm. Dans un premier temps, il a été noté que le détecteur indique zéro ppm d'ozone lorsque la poudre est encore à température ambiante.

Dans un second temps, la température de l'étuve est augmentée progressivement (en une heure environ) jusqu'à atteindre 40°C. Cette augmentation a pour objectif de faciliter le dégazage. L'évolution de la composition du ciel gazeux du réacteur est suivie avec le détecteur au cours du temps.

A température ambiante, le détecteur affichait 0 ppm. En revanche, l'augmentation de la température a provoqué un accroissement de la concentration d'ozone au cours du temps lue sur le détecteur (exemple : 0,12 ppm mesuré en 1h30). Ces expériences confirment donc que le matériau fabriqué selon la présente invention stocke bien de l'ozone, et que de l'ozone sous forme gazeuse est libérée lorsque le matériau est soumis à une température supérieure à la température ambiante.

### Conclusion :

Ces deux tests montrent que le matériau fabriqué selon la présente invention stocke bien de l'ozone, et permet de libérer de l'ozone gazeux lorsque ce matériau est dissous en partie dans un solvant (dans l'eau lors du premier test), et/ou est mis dans des conditions thermodynamiques (ici une température de 40°C sous 1 bar, dans le second test) défavorables au stockage d'ozone, rendant l'ozone moins stable dans le matériau et ainsi permettant dans ces conditions la libération d'une quantité mesurable d'O₃ dans le temps donné.

### II.4. Conclusions.

La mise en contact de l'ozone avec les cyclodextrines comme la β-CD et la HP-β-CD en phase solide conduit à l'obtention d'un matériau ayant des propriétés oxydatives puissantes. Les synthèses sont reproductibles ainsi que les résultats obtenus (dosages, efficacité biologique, etc). Le produit obtenu en fin de synthèse est une poudre fine facilement utilisable pour les applications visées (cf tests biologiques).

En faisant l'hypothèse que des molécules d'ozone sont encapsulées au sein du matériau, les concentrations d'ozone obtenues par dosage sont très élevées (de 100 à 1000 fois celles obtenues avec de l'eau ozonée). Cette concentration dépend de plusieurs paramètres, dont la nature et les propriétés de la CD.

La capacité de stockage du matériau dépend fortement de la CD utilisée comme matière première. Les meilleurs résultats ont été obtenus avec la HP-β-CD (une β-CD modifiée beaucoup plus soluble en phase aqueuse que la simple β-CD) pour laquelle il a été trouvé une concentration d'ozone égale à 5700 ± 100 µg/g de poudre (trois dosages effectués) avec l'exemple 1 de procédé et de 11540 ± 540 µg/g poudre (trois dosages effectués) avec l'exemple 2 de procédé (point I.2. ci-dessus). Ces valeurs équivalent à une concentration environ 400 fois et 800 fois plus forte que pour de l'eau ozonée à température et pression ambiantes ([O₃]eau ozonée, 25°C, 1 bar, à 60-80 g/Nm³ (pH = 7) environ 14 mg/L d'eau), respectivement.

Il a également été montré que le matériau maintient ses propriétés oxydatives durant plusieurs mois, même avec un stockage rudimentaire en conditions ambiantes (Température d'environ 21°C sous air). Néanmoins, la concentration d'ozone dans le matériau stocké en conditions ambiantes (Température d'environ 25-30°C et exemple 1 de procédé de synthèse) diminue au cours du temps : la perte estimée est d'environ 20% par jour si on considère le dosage « immédiat » de la poudre (première décoloration de la solution de KI). Les mesures réalisées avec des poudres synthétisées avec l'exemple 2 de procédé et stockées à différentes températures montrent que des températures basses de conservation (2°C, -19°C) permettent de limiter la perte d'ozone au cours du temps, les meilleurs résultats ayant été obtenus avec la température de stockage la plus basse testée l.e.-19°C)

Par ailleurs, les résultats de dosage pourraient également suggérer que le solide, une fois dissous dans l'eau, stabilise une partie de l'ozone, initialement contenue dans le matériau, au sein de la solution liquide qui se recolore progressivement au cours du temps. Les concentrations annoncées ainsi que les cinétiques pourraient être ainsi sous estimées par rapport à la réalité.

### Références

[1] McTurk & Waller, 1964, « Ozone carbon tetrachloride double hydrate », Nature, vol. 202, page 1107.
[2] Nakagima et al, 2012, « Molecular storage of ozone in a clathrate hydrate: an attempt for preserving ozone at high concentrations », PlosOne, vol. 7: e48563.
[3] Demande de brevet JP 2007/210881 au nom de Kurita Water Ind. Ltd., publiée le 23 août 2007.
[4] Dettmer et al, 2017, « Stabilization and prolonged reactivity of aqueousphase ozone wuth cyclodextrin », Journal of Contaminant Hydrology, vol. 196, pages 1-9.
[5] Demande de brevet US 2018/0178263 au nom de OXYTEC LLC, publiée le 28 juin 2018.
[6] Demande de brevet US 2016/0367967 au nom de Temple University of the Commonwealth System of Higher Education, publiée le 22 décembre 2016.
[7] Demande internationale WO 2006/134299 au nom de l'Université de Franche-Comté, publiée le 21 décembre 2006.

## Revendications

1. Procédé de préparation d'un matériau solide de stockage de l'ozone comprenant la mise en contact de cyclodextrines et/ou de dérivés de cyclodextrines se présentant sous forme solide avec un gaz comprenant de l'ozone, moyennant quoi un matériau solide de stockage de l'ozone est obtenu.

2. Procédé selon la revendication 1, **caractérisé en ce que** ledit dérivé de cyclodextrine est une cyclodextrine modifiée chimiquement, réticulée, immobilisée et/ou organisée en superstructure moléculaire.

3. Procédé selon la revendication 1 ou 2, **caractérisé en ce que** lesdites cyclodextrines et/ou lesdits dérivés de cyclodextrines sont choisis dans le groupe constitué par par les α-CD, les β-CD, les γ-CD, les α-CD hydroxypropylées, les β-CD hydroxypropylées, les γ-CD hydroxypropylées, les α-CD diméthylées, les β-CD diméthylées, les γ-CD diméthylées ; les sulfobutyléther-α-CD, les sulfobutyléther-β-CD, les sulfobutyléther-γ-CD, les α-CD sulfatées, les β-CD sulfatées, les γ-CD sulfatées, les α-CD phosphatées, les β-CD phosphatées, les γ-CD phosphatées ; les α-CD carboxyméthylées, les β-CD carboxyméthylées, les γ-CD carboxyméthylées, les α-CD carboxyméthyléthérées, les β-CD carboxyméthyléthérées, les γ-CD carboxyméthyléthérées, les 3-triméthylammonium-2-hydroxypropyl-éther-αCD ; les 3-triméthylammonium-2-hydroxypropyl-éther-β-CD ; les 3-triméthylammonium-2-hydroxypropyl-éther-γ-CD ; les dérivés de cyclodextrines réticulés et leurs mélanges.

4. Procédé selon l'une quelconque des revendications 1 à 3, **caractérisé en ce que** ledit procédé présente une étape préalable à la mise en contact entre lesdites cyclodextrines et/ou lesdits dérivés de cyclodextrines et ledit gaz comprenant de l'ozone visant soit à éliminer tout ou partie des molécules d'eau présentes dans les cavités desdites cyclodextrines et/ou desdits dérivés de cyclodextrine, soit à remplacer tout ou partie des molécules d'eau présentes dans les cavités des cyclodextrines et/ou des dérivés de cyclodextrines par une substance non réactive à l'ozone.

5. Procédé selon l'une quelconque des revendications 1 à 4, **caractérisé en ce que** ledit gaz comprenant de l'ozone est un mélange gazeux comprenant de l'ozone et au moins un autre gaz tel que du dioxygène, du dioxyde de carbone, du diazote ou un de leurs mélanges.

6. Procédé selon l'une quelconque des revendications 1 à 5, **caractérisé en ce que** la mise en contact entre lesdites cyclodextrines et/ou ledits dérivés de cyclodextrines et ledit gaz comprenant de l'ozone est réalisée à une température comprise entre 0°C et 80°C.

7. Procédé selon l'une quelconque des revendications 1 à 6, **caractérisé en ce que** la mise en contact entre lesdites cyclodextrines et/ou lesdits dérivés de cyclodextrines et ledit gaz comprenant de l'ozone dure entre 1 min et 8 h.

8. Installation susceptible d'être mise en œuvre dans le cadre d'un procédé de préparation tel que défini à l'une quelconque des revendications 1 à 7, ladite installation comprenant au moins un réacteur contenant des cyclodextrines et/ou des dérivés de cyclodextrines sous forme solide en connexion fluidique avec une source d'un gaz comprenant de l'ozone.

9. Installation selon la revendication 8, **caractérisée en ce que** ledit réacteur est un contacteur gaz/solide, opérant en lit fixe ou fluidisé, un mélangeur de poudre ou un réacteur agité et/ou **en ce que** ladite source d'ozone est un générateur d'ozone.

10. Installation selon la revendication 8 ou 9, **caractérisée en ce qu'**elle comprend, en outre, un ou plusieurs éléments choisi(s) dans le groupe constitué par un filtre, un destructeur d'ozone, un débitmètre, des sondes de température, des analyseurs d'ozone et des vannes.

11. Matériau solide de stockage de l'ozone susceptible d'être préparé par un procédé de préparation tel que défini à l'une quelconque des revendications 1 à 7.

12. Matériau solide de stockage de l'ozone selon la revendication 11, **caractérisé en ce qu'**il comprend des cyclodextrines et/ou des dérivés de cyclodextrines se présentant sous forme solide, dont au moins une partie des cavités contient de l'ozone.

13. Matériau solide de stockage de l'ozone selon la revendication 11 ou 12, ledit matériau se présentant sous forme compactée et/ou sous forme conditionnée.

14. Utilisation d'un matériau solide de stockage de l'ozone selon l'une quelconque des revendications 11 à 13 ou susceptible d'être préparé par un procédé de préparation tel que défini à l'une quelconque des revendications 1 à 7, comme agent désinfectant, dépolluant, nettoyant ou biocide.

15. Utilisation d'un matériau solide de stockage de l'ozone selon l'une quelconque des revendications 11 à 13 ou susceptible d'être préparé par un procédé de préparation tel que défini à l'une quelconque des revendications 1 à 7, pour désinfecter, dépolluer ou nettoyer un fluide ou une surface.

16. Utilisation selon la revendication 15, **caractérisée en ce que** ledit fluide est choisi parmi l'air ambiant ou l'atmosphère gazeuse d'un site tel qu'une pièce domestique, une chambre froide ou un espace confiné industriel ; de l'eau de ville, de rivière, de puits, de nappe phréatique, d'étang, de lac, de piscine, d'un aquarium, de refroidissement des systèmes de climatisation ou de tours aéro-réfrigérées ; un prélèvement dans un réacteur chimique ; une eau usée domestique ; un produit notamment liquide, un effluent ou de l'eau usée provenant notamment d'élevages intensifs ou d'industries ou d'installations du domaine chimique, pharmaceutique, cosmétique, agricole, agroalimentaire, maritime, aéronautique ou spatial ; ou un de leurs mélanges.

17. Utilisation selon la revendication 16, **caractérisée en ce que** ladite surface est choisie parmi un objet industriel comme un appareil électronique ou une machine utilisée dans l'agro-alimentaire, un véhicule, une carcasse, un aéronef, une cuve, une cuisine de restaurant, une chambre froide, un sanitaire, un conteneur, une partie d'une habitation comme un toit, une façade, une terrasse, une allée, des système embarqués dans l'espace, dans des bateaux ou dans des sous-marins, des dispositifs médicaux, des tuyaux, un sol ou de la terre, du bois et une surface végétale.

18. Matériau solide de stockage de l'ozone selon l'une quelconque des revendications 11 à 13 ou susceptible d'être préparé par un procédé de préparation tel que défini à l'une quelconque des revendications 1 à 7 pour utilisation comme médicament.

19. Utilisation d'un matériau solide de stockage de l'ozone selon l'une quelconque des revendications 11 à 13 ou susceptible d'être préparé par un procédé de préparation tel que défini à l'une quelconque des revendications 1 à 7 comme un réactif chimique.

## Patentansprüche

1. Verfahren zur Bereitung eines festen Ozonspeichermaterials, das das Inkontaktbringen von Cyclodextrinen und/oder Cyclodextrinderivaten in fester Form mit einem Ozon umfassenden Gas umfasst, wodurch ein festes Ozonspeichermaterial erhalten wird.

2. Verfahren nach Anspruch 1, **dadurch gekennzeichnet, dass** das besagte Cyclodextrinderivat ein chemisch modifiziertes, vernetztes, immobilisiertes und/oder in einer molekularen Überstruktur organisiertes Cyclodextrin ist.

3. Verfahren nach Anspruch 1 oder 2, **dadurch gekennzeichnet, dass** die besagten Cyclodextrine und/oder die besagten Cyclodextrinderivate ausgewählt sind aus der Gruppe bestehend aus α-Cyclodextrinen, β-Cyclodextrinen, γ-Cydodextrinen, hydroxypropylierten α-Cyclodextrinen, hydroxypropylierten β-Cyclodextrinen, hydroxypropylierten γ-Cydodextrinen,dimethylierten α-Cyclodextrinen, dimethylierten β-Cyclodextrinen, dimethylierten γ-Cyclodextrinen; Sulfobutylether-α-Cyclodextrinen, Sulfobutylether-β-Cyclodextrinen, Sulfobutylether-γ-Cyclodextrinen, sulfatierten α-Cyclodextrinen, sulfatierten β-Cyclodextrinen, sulfatierten γ-Cyclodextrinen, phosphatierten α-Cyclodextrinen, phosphatierten β-Cyclodextrinen, phosphatierten γ-Cydodextrinen; carboxymethylierten α-Cyclodextrinen, carboxymethylierten β-Cyclodextrinen, carboxymethylierten γ-Cydodextrinen, carboxymethyletherisierten α-Cyclodextrinen, carboxymethyletherisierten β-Cyclodextrinen carboxymethyletherisierten γ-Cydodextrinen,3-Trimethylammonium-2-hydroxypropylether-α-Cyclodextrinen, 3-Trimethylammonium-2-hydroxypropylether-ß-Cyclodextrinen 3-Trimethylammonium-2-hydroxypropylether-γ-Cyclodextrinen; vernetzten Cyclodextrinderivaten und Gemischen davon.

4. Verfahren nach einem der Ansprüche 1 bis 3, **dadurch gekennzeichnet, dass** das Verfahren einen Schritt vor dem Inkontaktbringen der besagten Cyclodextrine und/oder der besagten Cyclodextrinderivate mit dem besagten Ozon umfassenden Gas aufweist, der entweder darauf abzielt, die in den Hohlräumen der besagten Cyclodextrine und/oder der besagten Cyclodextrinderivate vorhandenen Wassermoleküle ganz oder teilweise zu entfernen, oder die in den Hohlräumen der Cyclodextrine und/oder der Cyclodextrinderivate vorhandenen Wassermoleküle ganz oder teilweise durch eine mit Ozon nicht reaktive Substanz zu ersetzen.

5. Verfahren nach einem der Ansprüche 1 bis 4, **dadurch gekennzeichnet, dass** das besagte Ozon umfassende Gas ein Gasgemisch ist, das Ozon und mindestens ein anderes Gas wie molekularen Sauerstoff, Kohlendioxid, molekularen Stickstoff oder ein Gemisch davon umfasst.

6. Verfahren nach einem der Ansprüche 1 bis 5, **dadurch gekennzeichnet, dass** das Inkontaktbringen der besagten Cyclodextrinen und/oder den besagten Cyclodextrinderivaten mit dem besagten Ozon umfassenden Gas bei einer Temperatur zwischen 0 °C und 80 °C durchgeführt wird.

7. Verfahren nach einem der Ansprüche 1 bis 6, **dadurch gekennzeichnet, dass** das Inkontaktbringen der besagten Cyclodextrinen und/oder der besagten Cyclodextrinderivaten mit dem besagten Ozon umfassenden Gas zwischen 1 min und 8 h dauert.

8. Anlage, die im Rahmen eines Bereitungsverfahrens nach einem der Ansprüche 1 bis 7 umgesetzt werden kann, wobei besagte Anlage zumindest einen Reaktor umfasst, der Cyclodextrine und/oder Cyclodextrinderivate in fester Form in Fluidverbindung mit einer Quelle eines Ozon umfassenden Gases enthält.

9. Anlage nach Anspruch 8, **dadurch gekennzeichnet, dass** besagter Reaktor ein Gas-/Feststoff-Kontaktor, der im Fest- oder Wirbelbett arbeitet, ein Pulvermischer oder ein Rührreaktor ist und/oder dass die Ozonquelle ein Ozongenerator ist.

10. Anlage nach Anspruch 8 oder 9, **dadurch gekennzeichnet, dass** sie ferner ein oder mehrere Elemente umfasst, die aus der Gruppe ausgewählt sind, die aus einem Filter, einem Ozonzerstörer, einem Durchflussmesser, Temperaturfühlern, Ozonanalysatoren und Ventilen besteht.

11. Festes Ozonspeichermaterial, das durch ein Bereitungsverfahren nach einem der Ansprüche 1 bis 7 bereitet werden kann.

12. Festes Ozonspeichermaterial nach Anspruch 11, **dadurch gekennzeichnet, dass** es Cyclodextrine und/oder Cyclodextrinderivate in fester Form umfasst, von denen zumindest ein Teil der Hohlräume Ozon enthält.

13. Festes Ozonspeichermaterial nach Anspruch 11 oder 12, wobei das besagte Material in verdichteter und/oder verpackter Form vorliegt.

14. Verwendung eines festen Ozonspeichermaterials nach einem der Ansprüche 11 bis 13 oder das durch ein Bereitungsverfahren nach einem der Ansprüche 1 bis 7 bereitet werden kann als Desinfektionsmittel, Entseuchungsmittel, Reinigungsmittel oder Biozid.

15. Verwendung eines festen Ozonspeichermaterials nach einem der Ansprüche 11 bis 13 oder das durch ein Bereitungsverfahren nach einem der Ansprüche 1 bis 7 hergestellt werden kann zum Desinfizieren, Entseuchen oder Reinigen eines Fluids oder einer Fläche.

16. Verwendung nach Anspruch 15, **dadurch gekennzeichnet, dass** das besagte Fluid ausgewählt ist aus der Umgebungsluft oder der Gasatmosphäre eines Ortes wie eines Hausraums, eines Kühlraums oder eines geschlossenen industriellen Raums; Stadt-, Fluss-, Brunnen-, Grundwasser, Teich-, See-, Schwimmbad-, Aquariumwasser, Kühlwasser von Klimaanlagen oder luftgekühlten Türmen; einer Entnahme aus einem chemischen Reaktor; einem Haushaltsabwasser; einem insbesondere flüssigen Produkt, einem Abfluss oder Abwasser, insbesondere aus Intensivtierhaltungen oder aus Industrien oder Anlagen des chemischen, pharmazeutischen, kosmetischen, landwirtschaftlichen, Lebensmittel-, See-, Luftoder Raumfahrtbereichs; oder einem Gemisch davon.

17. Verwendung nach Anspruch 16, **dadurch gekennzeichnet, dass** die besagte Fläche ausgewählt ist aus einem industriellen Objekt wie einem elektronischen Gerät oder einer Maschine, die in der Lebensmittelindustrie verwendet wird, einem Fahrzeug, einem Gerippe, einem Flugzeug, einem Tank, einer Restaurantküche, einem Kühlraum, einem Sanitärraum, einem Behälter, einem Teil eines Hauses wie einem Dach, einer Fassade, einer Terrasse, einer Auffahrt, Systemen, die in den Raumfahrteinrichtungen, in Booten oder U-Booten integriert sind, medizinischen Geräten, Rohren, Boden oder Erde, Holz und einer Pflanzenoberfläche.

18. Festes Ozonspeichermaterial nach einem der Ansprüche 11 bis 13 oder das durch ein Bereitungsverfahren nach einem der Ansprüche 1 bis 7 bereitet werden kann zur Verwendung als Arzneimittel.

19. Verwendung eines festen Ozonspeichermaterials nach einem der Ansprüche 11 bis 13 oder das durch ein Bereitungsverfahren nach einem der Ansprüche 1 bis 7 hergestellt werden kann als chemisches Reagens.

## Claims

1. A process for preparing a solid ozone storage material comprising contacting cyclodextrins and/or cyclodextrin derivatives in solid form with a gas comprising ozone, whereby a solid ozone storage material is obtained.

2. The process according to claim 1, **characterised in that** said cyclodextrin derivative is a chemically modified, cross-linked, immobilized cyclodextrin and/or a cyclodextrin organised in a molecular superstructure.

3. The process according to claim 1 or 2, **characterised in that** said cyclodextrins and/or said cyclodextrin derivatives are selected from the group consisting of α-CDs, β-CDs, γ-CDs, hydroxypropylated α-CDs, hydroxypropylated β-CDs, hydroxypropylated γ-CDs, dimethylated α-CDs, dimethylated β-CDs, dimethylated γ-CDs; sulfobutylether-α-CDs, sulfobutylether-β-CDs, sulfobutylether-γ-CDs, sulfated α-CDs, sulfated β-CDs, sulfated γ-CDs, phosphated α-CDs, phosphated β-CDs, phosphated γ-CDs; carboxymethylated α-CDs, carboxymethylated β-CDs, carboxymethylated γ-CDs, carboxymethylether α-CDs, carboxymethylether β-CDs, carboxymethylether γ-CDs, 3-trimethylammonium-2-hydroxypropyl-ether-α-CDs; 3-trimethylammonium-2-hydroxypropyl-ether-p-CDs; 3-trimethylammonium-2-hydroxypropyl-ether-γ-CDs; cross-linked cyclodextrin derivatives and mixtures thereof.

4. The process according to any one of claims 1 to 3, **characterised in that** said process has a step prior to contacting said cyclodextrins and/or said cyclodextrin derivatives with said gas comprising ozone aiming at either removing all or part of water molecules present in the cavities of said cyclodextrins and/or said cyclodextrin derivatives, or replacing all or part of water molecules present in the cavities of the cyclodextrins and/or cyclodextrin derivatives with a non-ozone reactive substance.

5. The process according to any one of claims 1 to 4, **characterised in that** said gas comprising ozone is a gas mixture comprising ozone and at least one other gas such as dioxygen, carbon dioxide, nitrogen or a mixture thereof.

6. The process according to any one of claims 1 to 5, **characterised in that** contacting between said cyclodextrins and/or said cyclodextrin derivatives and said gas comprising ozone is carried out at a temperature between 0°C and 80°C.

7. The process according to any one of claims 1 to 6, **characterised in that** contacting between said cyclodextrins and/or said cyclodextrin derivatives and said gas comprising ozone lasts between 1 min and 8 h.

8. An installation that can be used in a preparation process as defined in any one of claims 1 to 7, said installation comprising at least one reactor containing cyclodextrins and/or cyclodextrin derivatives in solid form in fluid connection with a source of a gas comprising ozone.

9. The installation according to claim 8, **characterised in that** said reactor is a gas/solid contactor, operating in a fixed or fluidized bed, a powder mixer or a stirred reactor and/or **in that** said ozone source is an ozone generator.

10. The installation according to claim 8 or 9, **characterised in that** it further comprises one or more elements selected from the group consisting of a filter, an ozone scavenger, a flow meter, temperature probes, ozone analysers and valves.

11. A solid ozone storage that can be prepared by a preparation process as defined in any one of claims 1 to 7.

12. The solid ozone storage material according to claim 11, **characterised in that** it comprises cyclodextrins and/or cyclodextrin derivatives in solid form, at least some of the cavities of which contain ozone.

13. The solid ozone storage material according to claim 11 or 12, said material being in compacted form and/or in packaged form.

14. A use of a solid ozone storage material according to any one of claims 11 to 13 or that can be prepared by a preparation process as defined in any one of claims 1 to 7, as a disinfectant, depollutant, cleaner or biocide agent.

15. A use of a solid ozone storage material according to any one of claims 11 to 13 or that can be prepared by a preparation process as defined in any one of claims 1 to 7, for disinfecting, depolluting or cleaning a fluid or a surface.

16. The use according to claim 15, **characterised in that** said fluid is selected from the ambient air or gaseous atmosphere of a site such as a domestic room, a cold room or an industrial confined space; city water, river water, well water, ground water, pond water, lake water, swimming pool water, aquarium water, cooling water from air conditioning systems or cooling towers; a sample from a chemical reactor; domestic waste water; a product, especially a liquid, effluent or waste water from intensive livestock farming or from industries or facilities in the chemical, pharmaceutical, cosmetic, agricultural, agri-food, maritime, aeronautical or space sectors; or a mixture thereof.

17. The use according to claim 16, **characterised in that** said surface is selected from an industrial object such as an electronic device or a machine used in the agri-food industry, a vehicle, a carcass, an aircraft, a tank, a restaurant kitchen, a cold room, a sanitary facility, a container, a part of a dwelling such as a roof, a facade, a terrace, a driveway, systems embarked in space, in ships or in submarines, medical devices, pipes, soil or earth, wood and a plant surface.

18. The solid ozone storage material according to any one of claims 11 to 13 or that can be prepared by a preparation process as defined in any one of claims 1 to 7 for use as a medicine.

19. A use of a solid ozone storage material according to any one of claims 11 to 13 or that can be prepared by a preparation process as defined in any one of claims 1 to 7 as a chemical reagent.
